# FASCICULE DE BREVET EUROPEEN

(11) **EP 2 880 051 B1**
(45) Date de publication et mention de la délivrance du brevet: **09.10.2019**
(21) Numéro de dépôt: 13801610.0
(22) Date de dépôt: 02.08.2013
(51) Int. Cl.: C07K 14/44, A61K 39/002, A61K 39/00

(54) **SOUCHES MUTANTES DE NEOSPORA ET LEURS UTILISATIONS**
MUTANTENSTÄMME VON NEOSPORA UND VERWENDUNGEN DAVON
MUTANT STRAINS OF NEOSPORA AND USES THEREOF

(30) Priorité: 02.08.2012 FR 1257544
(43) Date de publication de la demande: 10.06.2015
(73) Titulaire: Vitamfero, 37200 Tours (FR)
(72) Inventeur: BOUSSEMART-PROUVOST, Anne-France, F-37200 Tours (FR); BRETON, Pascal, F-37200 Tours (FR); LAMARQUE, Mauld, F-37200 Tours (FR); MORISSE-PHILIPPE, Solen, F-37200 Tours (FR); SECHE, Edouard, F-37200 Tours (FR)
(74) Mandataire: Grosset-Fournier, Chantal Catherine
(86) Numéro de dépôt international: PCT/FR2013/051877
(87) Numéro de publication internationale: WO 2014/020291

(56) Documents cités:
- EP-A2- 0 953 641
- WO-A1-2005/072754
- DIANA MARCELA PENARETE-VARGAS ET AL: "Protection against Lethal Neospora caninum Infection in Mice Induced by Heterologous Vaccination with a mic1 mic3 Knockout Toxoplasma gondii Strain", INFECTION AND IMMUNITY, AMERICAN SOCIETY FOR MICROBIOLOGY, US, vol. 78, no. 2, février 2010 (2010-02), pages 651-660, XP002696799, ISSN: 0019-9567, DOI: 10.1128/IAI.00703-09 [extrait le 2009-12-07]
- A. NAGULESWARAN ET AL: "Neospora caninum Microneme Protein NcMIC3: Secretion, Subcellular Localization, and Functional Involvement in Host Cell Interaction", INFECTION AND IMMUNITY, vol. 69, no. 10, octobre 2001 (2001-10), pages 6483-6494, XP055061688, ISSN: 0019-9567, DOI: 10.1128/IAI.69.10.6483-6494.2001
- JINHUA DONG ET AL: "Development of a diagnostic method for neosporosis in cattle using recombinant Neospora caninum proteins", BMC BIOTECHNOLOGY, BIOMED CENTRAL LTD. LONDON, GB, vol. 12, no. 1, 4 May 2012 (2012-05-04), page 19, XP021115716, ISSN: 1472-6750, DOI: 10.1186/1472-6750-12-19

## Description

La présente invention concerne une souche mutante de *Neospora spp*, son utilisation dans une composition pharmaceutique et son utilisation dans une composition vaccinale.

*Neospora caninum* est un parasite intracellulaire, responsable de la néosporose. Il appartient au phylum des Apicomplexes (embranchement des Apicomplexa) qui regroupe un grand nombre de parasites majoritairement intracellulaires. Ces parasites sont responsables de maladies telles que la néosporose, la toxoplasmose, le paludisme, la coccidiose et la cryptosporidiose. Ils ont en commun un processus spécifique d'invasion de cellules hôtes en plusieurs étapes conduisant à la formation d'une vacuole parasitophore dans laquelle le parasite se développe.

Le cycle de vie de *Neospora caninum* présente deux phases distinctes : une phase asexuée chez un hôte intermédiaire tel que la souris, les ovins et les bovins qui conduit à la production de tachyzoïtes puis de kystes contenant les bradyzoïtes, et une phase sexuée chez l'hôte définitif (principalement le chien) qui conduit à la production d'oocystes, contenant des sporozoïtes, éliminés dans les fèces.

La néosporose animale pose un problème économique important dans le domaine de l'élevage agricole et notamment dans les élevages bovins où elle provoque une diminution de la prise de poids des veaux, une diminution de la fertilité, une diminution de la production laitière mais est surtout reconnue comme étant l'une des causes majeures d'avortements. Ainsi, chaque année dans le Monde, 30 à 40 millions d'avortements sont provoqués par *Neospora caninum* dans les cheptels bovins. Contrairement à *Toxoplasma gondii*, la transmission materno-foetale du parasite et l'infection congénitale du foetus interviennent non seulement en cas de primo-infection au cours de la gestation mais également chez les vaches infectées de manière chronique préalablement à la gestation.

La contamination des bovins peut se faire selon deux voies bien distinctes :
- Par contamination horizontale c'est-à-dire par ingestion d'aliments contaminés par des oocystes excrétés par un hôte définitif. En cas de gestation, le risque d'avortement est alors de 80%.
- Par contamination verticale c'est-à-dire par transmission materno-foetale du parasite de la mère à ses veaux. Une vache infectée préalablement à la gestation, peut au cours des gestations suivantes, soit de nouveau avorter, soit donner naissance à un veau sain, soit, le plus souvent, transmettre le parasite à son veau. Ce veau sera infecté par *Neospora caninum* et si, il s'agit d'une femelle, elle aura, à son tour, un risque élevé de transmettre le parasite à sa descendance avec possibilité d'avortement.

Ces conséquences ont bien évidemment des répercussions économiques importantes pour les élevages. Ainsi, la néosporose est responsable d'une perte de 35 M€ pour 1,2 million de vaches laitières en Californie (Dubey, J. Am. Vet. Med. Assoc., 1999, Apr 15; 214(8):1160-3), de 19 M€ pour 1,6 million de vaches aux Pays-Bas (Bartels et al., Vet. Parasitol., 2006, Apr 15; 137(1-2):17-27) et de 10 M€ pour 0,7 million de vaches en Suisse (Häsler et al., Prev. Vet. Med., 2006, Dec 18;77(3-4):230-53).

Le développement d'un vaccin constitue un objectif majeur pour lutter contre la néosporose. Plusieurs stratégies pour construire un vaccin contre la néosporose sont actuellement à l'étude :
- Les vaccins basés sur des parasites inactivés par irradiation, par chaleur, *etc.* Ces vaccins génèrent une réponse immunitaire protectrice mais nécessitent généralement la présence d'adjuvants et également de nombreux rappels de vaccination. Actuellement, un seul vaccin dirigé contre la néosporose bovine est commercialisé dans certaines zones géographiques et notamment aux Etats-Unis. Il s'agit du vaccin NeoGuard® commercialisé par la société Intervet. Ce vaccin est constitué de tachyzoïtes inactivés de *Neospora caninum* et d'un adjuvant particulier (Havlogene). L'efficacité du vaccin NeoGuard®, d'environ 50%, est considérée comme partielle. Par ailleurs, le mode d'administration de ce vaccin nécessite deux injections à quelques semaines d'intervalle lors de la première année de vaccination puis une à deux injections les années suivantes.
- Les vaccins basés sur des parasites vivants atténués ; ces souches sont généralement obtenues soit *in vitro* par passages successifs du parasite en culture dans des cellules hôtes en présence ou non d'agents mutagènes, soit par l'isolement dans la nature de souches moins virulentes, soit par d'autres procédés tels que l'irradiation. Ces vaccins sont généralement très efficaces mais présentent l'inconvénient majeur de ne pas être caractérisés sur le plan moléculaire et des retours vers une forme virulente sont toujours à craindre. Plusieurs isolats tels que Nc-Nowra (Miller et al., Aust. Vet. J., 2002, Oct; 80(10):620-5) et Nc-Spain-1H (Regidor-Cerrillo et al., Parasitology, 2008, Dec; 135(14): 1651-9) ont été isolés à partir de veaux infectés de manière asymptomatique. Le potentiel vaccinal de ces souches atténuées est en cours d'évaluation.
- Les vaccins recombinants : un virus de la vaccine exprimant l'antigène NcSRS2 de *N. caninum* a été évalué dans des expériences de vaccination chez la souris (Nishikawa et al., Parasitol. Res., 2000, Nov;86(11):934-9 ; Nishikawa et al., Vaccine, 2001, Jan 8; 19(11-12): 1381-90) et a démontré sa capacité à induire une réponse immunitaire protectrice chez la souris. Plus récemment, une souche de *Brucella abortus* a été utilisée pour exprimer différents antigènes de *N. caninum* (Ramamoorthy et al., Int. J. Parasitol., 2007, Nov;37(13):1531-8 and Ramamoorthy et al., Int J Parasitol., 2007 Nov;37(13):1521-9). Les souches exprimant la protéine NcMIC1 ou la protéine NcGRA6 protègent efficacement les souris d'une infection à *N. caninum* mais soulèvent la problématique de l'utilisation d'une souche pathogène chez le bovin.
- Les vaccins sub-unitaires composés de protéines issues des parasites capables d'induire une réponse immunitaire protectrice. Ces vaccins nécessitent généralement la présence non seulement d'adjuvants mais également de nombreux rappels de vaccination et sont généralement moins efficaces en terme de réponse immune induite chez l'individu vacciné. Plusieurs essais de vaccination ont été entrepris avec différentes protéines antigéniques. Ainsi, l'inoculation de la protéine NcSRS2 à des souris bloque la transmission materno-foetale (Haldorson et al., Int. J. Parasitol., 2005, Nov;35(13):1407-15) et associée à l'adjuvant de Freund, induit une réponse immunitaire proche de la réponse immunitaire induite par l'inoculation de tachyzoïtes *N. caninum* vivants (Staska et al., Infect. Immun., 2005, Mar;73(3):1321-9). Des essais de vaccination ont également été entrepris chez la souris avec la protéine NcMIC1 ou NcMIC3 et ont démontré une prévention des infections cérébrales après un challenge infectieux (Cannas et al., J. Parasitol., 2003, Feb;89(1):44-50 ; Allaedine et al., J. Parasitol., 2005, Jun;91(3):657-65). Il a aussi été démontré que des anticorps de souris immunisées avec la protéine Nc-AMA1 réduisaient significativement l'infection cellulaire de *N. caninum.*

Malgré des conséquences économiques fortes dans les élevages bovins, aucun vaccin efficace, sûr et d'utilisation simple n'est actuellement commercialisé ou en phase de développement. Il existe, en conséquence, un réel besoin de mettre à disposition un vaccin à la fois efficace contre la néosporose, facile à utiliser et présentant une excellente innocuité.

De manière surprenante, les inventeurs ont trouvé que la suppression de la fonction de la protéine NcMIC3 seule, ou la suppression de la fonction des deux protéines NcMIC3 et NcMIC1, dans une souche de *Neospora caninum*, conduit à une souche mutante qui possède des propriétés infectieuses et immunogènes conférant aux mammifères une protection vaccinale vis-à-vis des effets délétères de la néosporose.

La présente description divulgue une souche mutante de *Neospora spp* dans laquelle la fonction de la protéine NcMIC3 est supprimée.

La présente invention a donc pour objet une souche mutante de *Neospora spp* dans laquelle la fonction de la protéine NcMIC3 et, éventuellement, la fonction de la protéine NcMIC1, est supprimée.

La présente invention a donc pour objet une souche mutante de *Neospora spp* dans laquelle la fonction de la protéine NcMIC3 est supprimée, notamment par une inhibition de l'expression du gène *ncmic3*, et éventuellement la fonction de la protéine NcMIC1 est supprimée, notamment par une inhibition de l'expression du gène *ncmic1.*

Les protéines sont les effecteurs de l'activité cellulaire. La suppression de la fonction d'une protéine peut résulter de son absence de biosynthèse ou de sa non fonctionnalité. L'origine de ce dysfonctionnement peut être liée à des perturbations survenant lors de la transcription du gène codant la protéine, lors de sa traduction ou encore survenir lors de processus de maturation de la protéine (modifications post-traductionelles). La délétion du gène explique également qu'aucune protéine ne puisse être synthétisée.

Les protéines NcMIC1 et NcMIC3 sont des protéines des micronèmes, organelles sécrétoires des Apicomplexes qui jouent un rôle central dans la reconnaissance et l'adhésion aux cellules hôtes. Chez *Neospora caninum,* la protéine NcMIC1 est une protéine de 460 acides aminés codée par le gène *ncmic1* qui comporte 4 exons. La séquence polypeptidique de NcMIC1 contient un peptide signal de 20 acides aminés suivi par deux régions de répétition (48 acides aminés et 44 acides aminés) en tandem (Keller et al., Infect Immun. 2002 Jun;70(6):3187-98).

La protéine NcMIC3 de *Neospora caninum* est codée par le gène *ncmic3* qui comporte un seul exon. Cette protéine possède 362 acides aminés.

Les Inventeurs ont construit une souche mutante de *Neospora caninum* appelée Neo *ncmic1-3* KO, dans laquelle la fonction de la protéine NcMIC1 et la fonction de la protéine NcMIC3 ont été supprimées, une souche mutante Neo *ncmic3* KO, dans laquelle seule la fonction de la protéine NcMIC3 a été supprimée et, une souche mutante Neo *ncmic1* KO, dans laquelle seule la fonction de la protéine NcMIC1 a été supprimée. Ces trois souches mutantes de *Neospora caninum* sont le premier exemple de souches vivantes atténuées de *Neospora caninum* obtenues par la délétion ciblée et contrôlée de gènes de virulence ou par la suppression ciblée et contrôlée des fonctions de protéines de virulence.

Dans la présente invention, on entend par « la fonction de la protéine NcMIC1 est supprimée », soit l'absence de l'expression de la protéine NcMIC1, soit l'expression d'une protéine NcMIC1 non fonctionnelle, par exemple l'expression d'une protéine ne possédant pas la fonction de la protéine NcMIC1 et ayant une certaine identité de séquence en acides aminés avec celle de la protéine NcMIC1.

On entend par « la fonction de la protéine NcMIC3 est supprimée », soit l'absence de l'expression de NcMIC3, soit l'expression d'une protéine NcMIC3 non fonctionnelle, par exemple une protéine ne possédant pas la fonction de la protéine NcMIC3 et ayant une certaine identité de séquence en acides aminés avec celle de la protéine NcMIC3.

L'absence de l'expression de la protéine NcMIC1 ou de la protéine NcMIC3 peut résulter de la délétion de la totalité du gène *ncmic1* ou *ncmic3*, ou de sa région codante, ou d'une mutation, d'une délétion ou d'une insertion d'un ou plusieurs nucléotides dans la région codante du gène *ncmic1* ou *ncmic3* entrainant l'absence d'expression des protéines ou des protéines avec peu d'identité de séquence en acides aminés avec les protéines NcMIC1 ou NcMIC3, ou d'un dysfonctionnement de la région promotrice ou de la région régulatrice en *cis* ou en *trans* du gène *ncmic1* ou *ncmic3*, ou d'un dysfonctionnement d'un ou plusieurs facteurs de transcription susceptibles de se lier à ladite région promotrice, ou d'un dysfonctionnement de la traduction de l'ARN messager, ou de quelques modifications épigénétiques bien connues de l'homme de l'art. Ainsi, on entend par « inhibition de l'expression du gène *ncmic1* ou *ncmic3* », tous les mécanismes qui perturbent la transcription du gène *ncmic1* ou *ncmic3* en un ARN messager ou tous les mécanismes qui perturbent la traduction des ARN messager en protéines NcMIC1 ou NcMIC3, ces deux étapes étant nécessaires à la synthèse d'une protéine NcMIC1 ou NcMIC3 fonctionnelle.

Une protéine NcMIC1 non fonctionnelle ou une protéine NcMIC3 non fonctionnelle est une protéine qui ne possède pas la capacité de reconnaître les cellules hôtes ou qui ne permet pas l'adhésion du parasite audites cellules hôtes. Une protéine non fonctionnelle peut résulter d'une mutation, d'une délétion ou d'une insertion d'un ou plusieurs nucléotides dans la région codante du gène *ncmic1* ou *ncmic3.* Dans ce cas de figure, la modification de l'acide nucléique de la région codante ne bloque pas le mécanisme d'expression de la protéine qui peut éventuellement conserver une certaine identité de séquence en acides aminés avec celle de la protéine NcMIC1 ou celle de la protéine NcMIC3, mais change le cadre de lecture de l'ARNm correspondant lors de la traduction de la protéine. La non fonctionnalité de la protéine NcMIC3, ou de la protéine NcMIC1, peut également être la conséquence de modifications post-traductionnelles inopérantes ou insuffisantes (*i.e*. glycosylation, isoprénylation, phosphorylation, sulfatation, amidation, acétylation, alkylation, *etc.*) et qui lui permettaient d'assurer sa fonction.

La fonction de la protéine NcMIC3, ou de la protéine NcMIC1, peut également être supprimée de manière indirecte, notamment en altérant ou en supprimant l'expression d'une ou plusieurs autres protéines (notamment d'autres adhésines) qui se lient à la protéine NcMIC3, ou à la protéine NcMIC1, pour former un complexe fonctionnel. La déstructuration d'un tel complexe entraine une perte de fonction de la protéine NcMIC3, ou de la protéine NcMIC1.

Dans un mode de réalisation particulier, l'invention concerne une souche mutante de *Neospora* dans laquelle seule la fonction de la protéine NcMIC3 est supprimée.

Les Inventeurs ont constaté que la suppression de la fonction de la protéine NcMIC3 seule chez *Neospora caninum* permet de réduire significativement la virulence *in vivo* du parasite. Néanmoins, la double suppression de la fonction de la protéine NcMIC3 et de la fonction de la protéine NcMIC1 chez *Neospora caninum* accentue encore davantage l'atténuation de la virulence du parasite.

Dans un mode de réalisation particulier, l'invention concerne une souche mutante de *Neospora spp* dans laquelle la fonction de la protéine NcMIC3 et la fonction de la protéine NcMIC1 sont supprimées.

Dans un mode de réalisation particulier, l'invention concerne une souche mutante de *Neospora spp* dans laquelle à la fois la fonction de la protéine NcMIC3 et la fonction de la protéine NcMIC1 sont supprimées par une inhibition de l'expression des deux gènes *ncmic3* et *ncmic1.*

La fonction de la protéine NcMIC3 de la souche mutante de *Neospora spp* peut être supprimée par :
- une mutation, une délétion ou une insertion d'un ou plusieurs nucléotides dans la séquence nucléotidique du gène *ncmic3* ou dans une séquence nucléotidique qui conditionne l'expression de la protéine NcMIC3, ou
- une déstabilisation de l'ARN messager résultant de la transcription du gène *ncmic3,* ou
- une inhibition de la traduction de l'ARN messager du gène *ncmic3* ou d'une séquence nucléotidique qui conditionne l'expression de la protéine NcMIC3.

La fonction de la protéine NcMIC1 de la souche mutante de *Neospora spp* peut être supprimée par :
- une mutation, une délétion ou une insertion d'un ou plusieurs nucléotides dans la séquence nucléotidique du gène *ncmic1* ou dans une séquence nucléotidique qui conditionne l'expression de la protéine NcMIC1, ou
- une déstabilisation de l'ARN messager résultant de la transcription du gène *ncmic1*, ou
- une inhibition de la traduction de l'ARN messager du gène *ncmic1* ou d'une séquence nucléotidique qui conditionne l'expression de la protéine NcMIC1.

Par « mutation d'un ou plusieurs nucléotides », on entend la substitution, permutation ou le remplacement d'un ou plusieurs nucléotides d'une séquence nucléotidique par un ou plusieurs nucléotides non présents dans la séquence sauvage. Par séquence sauvage, on désigne la séquence nucléotidique trouvée à l'état naturel dans la souche sauvage du parasite. La séquence sauvage est par définition dépourvue de toute intervention humaine par le biais du génie génétique. Dans la présente invention, la souche sauvage de référence de *N. caninum* est la souche NC1.

Par « délétion d'un ou plusieurs nucléotides », on entend la suppression d'un ou plusieurs nucléotides d'une séquence nucléotidique.

Par « insertion d'un ou plusieurs nucléotides », on entend l'addition, l'ajout ou l'intégration d'un ou plusieurs nucléotides dans une séquence nucléotidique.

La mutation, la délétion ou l'insertion d'un ou plusieurs nucléotides peut avoir lieu à l'intérieur d'un ou plusieurs exons du gène correspondant et modifier par conséquent la région codante dudit gène, ou bien avoir lieu à l'intérieur d'un ou plusieurs introns et modifier le site d'épissage d'un intron concerné. Cette modification du site d'épissage change par conséquent le cadre de lecture de l'ARNm et aboutit à la traduction d'une nouvelle protéine dont la séquence en acides aminés diffère de la séquence de la protéine dite sauvage.

Par « déstabilisation de l'ARN messager », on entend une diminution de sa durée de demi-vie, c'est-à-dire de la période de temps pendant laquelle un ARN messager est disponible pour permettre sa traduction en une protéine. La stabilisation des ARN messagers est assurée par des éléments *cis* (les séquences 5' et 3' UTR bordant les séquences codantes d'un gène) et des éléments *trans*, notamment des protéines capables de se lier aux éléments *cis.* La durée de demi-vie d'un ARN messager peut varier en réponse à divers stimuli tels que des facteurs environnementaux, des facteurs de croissance ou des hormones. Une modification, réalisée *in vitro* par génie génétique, des séquences nucléotidiques des éléments *cis* est susceptible de modifier la durée de demi-vie de l'ARN messager.

Par « inhibition de la traduction de l'ARN messager d'un gène », on entend le blocage de la traduction de l'ARN messager en la protéine lui correspondant. Dans ce cas de figure, l'ARN messager d'un gène est présent dans la cellule, alors que la protéine lui correspondant est absente. L'inhibition de la traduction de l'ARN messager d'un gène peut résulter du dysfonctionnement d'un élément de la machinerie traductionnelle, notamment des ribosomes, des ARNs ribosomiques (ARNr) ou des ARNs de transfert (ARNt), ou des aminoacyl-ARNt synthétases.

Dans un mode de réalisation plus particulier, l'invention concerne une souche mutante de *Neospora spp*, dans laquelle :
- la fonction de la protéine NcMIC3 est supprimée par une mutation, une délétion ou une insertion d'un ou plusieurs nucléotides dans la séquence nucléotidique du gène *ncmic3*, et éventuellement
- la fonction de la protéine NcMIC1 est supprimée par une mutation, une délétion ou une insertion d'un ou plusieurs nucléotides dans la séquence nucléotidique du gène *ncmic1.*

Une telle mutation, délétion ou insertion d'un ou plusieurs nucléotides dans la séquence nucléotidique du gène *ncmic3* ou du gène *ncmic1* peut conduire à l'absence d'expression de la protéine NcMIC3 ou NcMIC1, ou à la production d'une protéine non fonctionnelle ayant ou non une certaine identité de séquence d'acides aminés avec celle de la protéine NcMIC3 ou NcMIC1.

Plus particulièrement, l'invention concerne une souche mutante de *Neospora spp,* dans laquelle à la fois la fonction de la protéine NcMIC3 et la fonction de la protéine NcMIC1 sont supprimées par une mutation, une délétion ou une insertion d'un ou plusieurs nucléotides dans les séquences nucléotidiques des gènes *ncmic3* et *ncmic1.*

Dans un autre mode de réalisation particulier, l'invention concerne une souche mutante de *Neospora spp*, dans laquelle :
- la fonction de la protéine NcMIC3 est supprimée par la délétion de tout ou partie du gène *ncmic3* ou de sa région promotrice, et éventuellement
- la fonction de la protéine NcMIC1 est supprimée par la délétion de tout ou partie du gène *ncmic1* ou de sa région promotrice.

On entend par « délétion du gène », la suppression du gène entier c'est-à-dire les introns et les exons, ou de la région codante entière du gène c'est-à-dire uniquement les exons. On entend par « région promotrice », la séquence nucléotidique située en amont de la région 5' UTR transcrite non traduite qui sert de boitier de régulation de l'expression d'un gène.

Plus particulièrement, l'invention concerne une souche mutante de *Neospora spp*, dans laquelle la fonction de la protéine NcMIC3 est supprimée par la délétion de tout ou partie du gène *ncmic3* ou de sa région promotrice et la fonction de la protéine NcMIC1 est supprimée par la délétion de tout ou partie du gène *ncmic1* ou de sa région promotrice.

Dans un autre mode de réalisation particulier, l'invention concerne une souche mutante de *Neospora spp*, dans laquelle :
- la fonction de la protéine NcMIC3 est supprimée par une mutation, une délétion ou une insertion d'un ou plusieurs nucléotides dans une séquence nucléotidique qui conditionne l'expression de la protéine NcMIC3, et éventuellement
- la fonction de la protéine NcMIC1 est supprimée par une mutation, une délétion ou une insertion d'un ou plusieurs nucléotides dans une séquence nucléotidique qui conditionne l'expression de la protéine NcMIC1.

Plus particulièrement, l'invention concerne une souche mutante de *Neospora spp*, dans laquelle
- la fonction de la protéine NcMIC3 est supprimée par une mutation, une délétion ou une insertion d'un ou plusieurs nucléotides dans une séquence nucléotidique qui conditionne l'expression de la protéine NcMIC3, et
- la fonction de la protéine NcMIC1 est supprimée par une mutation, une délétion ou une insertion d'un ou plusieurs nucléotides dans une séquence nucléotidique qui conditionne l'expression de la protéine NcMIC1.

Dans un mode de réalisation particulier, la souche mutante de *Neospora spp* selon la présente invention est une souche mutante de *Neospora caninum.*

La présente invention a également pour objectif de mettre à disposition une composition pharmaceutique comprenant une souche mutante de *Neospora* dans laquelle la fonction de la protéine NcMIC3, et éventuellement la fonction de la protéine NcMIC1, est supprimée.

Ladite composition pharmaceutique comprenant une souche mutante telle que décrite ci-dessus et un véhicule pharmaceutiquement acceptable.

Plus particulièrement, une telle composition pharmaceutique est administrée en une dose unitaire variant de 10² à 10⁹ tachyzoïtes d'une souche mutante de *Neospora spp.*

Plus particulièrement, une telle composition pharmaceutique est administrée en une dose unitaire variant de 10² à 10⁹ tachyzoïtes de la souche Neo *ncmic1-3* KO.

Encore plus particulièrement, une telle composition pharmaceutique est administrée en une dose unitaire variant de 10³ à 10⁸, notamment de 10⁴ à 10⁷, notamment de 10⁵ à 10⁶ tachyzoïtes de la souche Neo *ncmic1-3* KO.

Encore plus particulièrement, une telle composition pharmaceutique est administrée en une dose unitaire variant de 10² à 10⁸, notamment de 10² à 10⁷, notamment de 10² à 10⁶, notamment de 10² à 10⁵, notamment de 10² à 10⁴, notamment de 10² à 10³ tachyzoïtes de la souche Neo *ncmic1-3* KO.

Encore plus particulièrement, une telle composition pharmaceutique est administrée en une dose unitaire de 10², 10³, 10⁴, 10⁵, 10⁶, 10⁷, 10⁸, ou de 10⁹ tachyzoïtes de la souche Neo *ncmic1-3* KO.

Dans un mode de réalisation plus particulier, la première administration peut être suivie de rappels ultérieurs possibles, selon les doses unitaires indiquées précédemment.

Par ailleurs, la présente invention a pour objectif de fournir une composition vaccinale comprenant une souche mutante *Neospora spp* selon la présente invention et un véhicule pharmaceutiquement acceptable.

Une telle composition pharmaceutique ou composition vaccinale peut être administrée par voie parentérale (intraveineuse, sous-cutanée, intradermique, intramusculaire, et intrapéritonéale) ou par voie entérale.

Le choix d'un véhicule pharmaceutique acceptable contenu dans une telle composition pharmaceutique ou composition vaccinale peut être fait en fonction du mode d'administration envisagé sur la base des connaissances de l'homme du métier.

Une telle composition pharmaceutique ou vaccinale peut être utilisée pour le traitement de la néosporose de primo-infection, de réactivation ou de réinfection chez les animaux de compagnie, tels que les chiens et les chevaux, et les animaux de rentes, tels que les ovins, les caprins, les bovins, les porcins, les camélidés et les cervidés.

Plus particulièrement, une telle composition pharmaceutique ou vaccinale peut être utilisée pour le traitement de la néosporose de primo-infection, de réactivation ou de réinfection chez les animaux de compagnie, tels que les chiens et les chevaux, et les animaux de rentes, tels que les ovins, les caprins, les bovins, les porcins, les camélidés et les cervidés et notamment pour prévenir la transmission materno-foetale du parasite afin de réduire le nombre d'avortements mais également le risque de contamination verticale des mères à leur descendance.

Encore plus particulièrement, une telle composition pharmaceutique ou vaccinale peut être utilisée pour le traitement de la néosporose de primo-infection, de réactivation ou de réinfection chez les animaux de compagnie, tels que les chiens et les chevaux, et les animaux de rentes, tels que les ovins, les caprins, les bovins, les porcins, les camélidés et les cervidés et notamment pour prévenir la transmission materno-foetale du parasite afin de réduire le nombre d'avortements mais également le risque de contamination verticale des mères à leur descendance en cas d'infection au cours de la gestation (*i.e.* infection aigue) mais également préalablement à la gestation (*i.e.* infection chronique).

La description décrit également une méthode de diagnostic *in vitro* permettant de différencier les animaux vaccinés avec les souches mutantes Neo *ncmic1* KO, Neo *ncmic3* KO, Neo *ncmic1-3* KO et les animaux naturellement infectés par les souches sauvages de *N. caninum.* Ces tests diagnostic, dénommés DIVA, (Differentiating Infected from Vaccinated Animal) sont de plus en plus exigés par les instances réglementaires notamment à des fins de pharmacovigilance et d'études épidémiologiques mais également afin d'identifier les raisons éventuelles d'avortements survenus chez des animaux vaccinés.

La description décrit également un procédé *in vitro* de diagnostic différentiel pour discriminer un mammifère vacciné par les compositions de l'invention d'un mammifère non vacciné, ledit procédé comprenant une étape de :
- détermination de la concentration en anticorps anti-NcMIC1 et/ou anti-NcMIC3 et/ou anti-DHFR et/ou anti-CAT-GFP, et/ou,
- détermination de la concentration en antigène NcMIC1 et/ou NcMIC3 et/ou DHFR et/ou CAT-GFP,
- détermination du niveau d'expression des gènes *ncmic1*, *ncmic3*, *dhfr* et/ou *cat-gfp*, et/ou
- détermination de la présence ou de l'absence des gènes *ncmic1*, *ncmic3*, *dhfr* et/ou *cat-gfp*,
dans un échantillon biologique du susdit mammifère.

La description décrit également un procédé *in vitro* de diagnostic différentiel pour discriminer un mammifère vacciné par les compositions de l'invention d'un mammifère non vacciné, ledit procédé comprenant les étapes suivantes :
i) détermination de la concentration en anticorps anti-NcMIC1 et/ou anti-NcMIC3 et/ou anti-DHFR et/ou anti-CAT-GFP,
   et/ou,
   détermination de la concentration en antigène NcMIC1 et/ou NcMIC3 et/ou DHFR et/ou CAT-GFP,
ii) détermination du niveau d'expression des gènes *ncmic1*, *ncmic3*, *dhfr* et/ou *cat-gfp*, et/ou,
   détermination de la présence ou de l'absence des gènes *ncmic1*, *ncmic3*, *dhfr* et/ou *cat-gfp*,
dans un échantillon biologique du susdit mammifère.

La présente invention concerne également un procédé *in vitro* de diagnostic différentiel pour discriminer si un mammifère est un mammifère vacciné par une composition telle que définie selon l'invention, ou un mammifère non vacciné, ledit procédé comprenant les étapes suivantes :
i) détermination de la présence d'anticorps anti-DHFR et/ou anti-CAT-GFP, et/ou, détermination de la présence d'antigènes DHFR et/ou CAT-GFP, ou
ii) détermination du niveau d'expression des gènes *dhfr* et/ou *cat-gfp*, et/ou, détermination de la présence des gènes *dhfr* et/ou *cat-gfp*,
dans un échantillon biologique du susdit mammifère,
la détermination de la présence d'anticorps anti-DHFR et/ou anti-CAT-GFP, et/ou la détermination de la présence d'antigènes DHFR et/ou CAT-GFP, ou la détermination du niveau d'expression des gènes *dhfr* et/ou *cat-gfp*, et/ou la détermination de la présence des gènes *dhfr* et/ou *cat-gfp*, signifiant que ledit mammifère est un mammifère vacciné.

Selon un mode de réalisation particulier, le procédé de la description ou de l'invention peut être mis en oeuvre sur un échantillon biologique choisi parmi le groupe constitué par le sang et le sérum mais également certains tissus et organes tels que le placenta, le cerveau, les muscles, *etc.*

Les souches sauvages de *Neospora caninum* possèdent dans leur génome les gènes *ncmic1* et *ncmic3* et expriment les protéines NcMIC1 et NcMIC3.

Les souches mutantes de *Neospora caninum* telles que définies selon la présente invention possèdent respectivement :
- le gène *ncmic1* et le gène de sélection *dhfr* pour la souche mutante Neo *ncmic3* KO ; le gène de sélection *dhfr* est inséré par recombinaison homologue en lieu et place du gène *ncmic3* dans le génome de cette souche mutante. La souche mutante Neo *ncmic3* KO exprime les protéines NcMIC1 et DHFR et n'exprime pas la protéine NcMIC3,
- les gènes de sélection *dhfr* et *cat-gfp* pour la souche mutante Neo *ncmic1-3* KO ; les gènes de sélection *dhfr* et *cat-gfp* sont insérés par recombinaison homologue en lieu et place respectivement du gène *ncmic3* et *ncmic1* dans le génome de cette souche mutante. La souche mutante Neo *ncmic1-3* KO exprime les protéines DHFR et CAT-GFP et n'exprime pas les protéines NcMIC1 et NcMIC3.

Le diagnostic entre des animaux vaccinés par les souches mutantes de la description ou de l'invention et des animaux infectés par des souches sauvages de *N. caninum* peut être indirect et basé sur la détection et l'identification d'anticorps, ou direct et basé sur la détection de l'agent infectieux avec des technologies d'immunologie ou moléculaires.

La présente description divulgue également un procédé de mise en évidence, dans un échantillon biologique notamment choisi parmi le groupe constitué par le sang et le sérum issu d'un mammifère, d'un anticorps anti-NcMIC1 et/ou d'un anticorps anti-NcMIC3 et/ou d'un anticorps anti-DHFR et/ou d'un anticorps anti-CAT-GFP, ledit procédé comprenant l'étape suivante :
- détermination de la présence d'au moins un anticorps présent dans un échantillon biologique préalablement prélevé d'un mammifère par formation *in vitro* d'un complexe immun, ledit complexe immun étant constitué de la protéine NcMIC1 liée à l'anticorps anti-NcMIC1 ou de la protéine NcMIC3 liée à l'anticorps anti-NcMIC3 ou la protéine DHFR liée à l'anticorps anti-DHFR ou la protéine CAT-GFP liée à l'anticorps anti-CAT-GFP, par comparaison avec un échantillon biologique de référence.

Par « complexe immun », on désigne l'interaction physique entre un antigène et un anticorps spécifiquement dirigé contre cet antigène. Dans la présente invention, cette interaction est réalisée *in vitro* entre les protéines NcMIC1, NcMIC3, DHFR, CAT-GFP et les anticorps spécifiquement dirigés contre chacune de ces protéines.

Par « détection et l'identification d'anticorps » on entend la détection d'anticorps spécifiques des antigènes recherchés et présents dans le sérum des individus. La détection des anticorps est réalisée par des techniques d'ELISA indirect classique ou de compétition.

Les techniques d'ELISA indirect reposent sur l'utilisation d'antigènes fixés sur des supports solides. Le sérum des individus à diagnostiquer est déposé sur le support pour générer des interactions entre l'antigène fixé et les anticorps éventuellement présents dans le sérum des individus à diagnostiquer. Après lavage, l'interaction antigène-anticorps est mise en évidence en utilisant des anticorps secondaires marqués reconnaissant spécifiquement les anticorps conjugué anti-espèce. La mise en évidence d'anticorps dirigés contre les protéines DHFR et/ou CAT-GFP permettra de mettre en évidence une inoculation antérieure des souches mutantes à l'individu. Au contraire, la mise en évidence d'anticorps dirigés contre les protéines NcMIC1 et NcMIC3 mettra en évidence une contamination préalable de l'individu par une souche sauvage de *N. caninum.*

L'ELISA de compétition repose sur la compétition entre les anticorps présents éventuellement dans le sérum de l'individu à diagnostiquer et des anticorps présents dans un sérum de détection. Les antigènes sont fixés sur des supports solides. Le sérum des individus à diagnostiquer et le sérum de compétition sont déposés sur le support. La liaison spécifique de l'anticorps de détection est mise en évidence en utilisant un conjugué anti-espèce approprié et marqué. La présence éventuelle d'anticorps dans le sérum de l'individu à diagnostiquer génère une compétition avec les anticorps présents dans le sérum de détection et entraîne une diminution de la révélation. L'ELISA indirect avec les protéines DHFR et/ou CAT-GFP permettra de mettre en évidence l'inoculation des souches mutantes de l'invention à l'animal. L'ELISA indirect avec les protéines NcMIC1 et NcMIC3 permettra de mettre en évidence la contamination de l'animal par une souche sauvage de *N. caninum.*

La présente description divulgue également un procédé de mise en évidence, dans un échantillon biologique notamment choisi parmi le groupe constitué par le sang et le sérum mais également certains tissus et organes tels que le placenta, le cerveau, les muscles, *etc.* issu d'un mammifère, de l'antigène NcMIC1 et/ou de l'antigène NcMIC3 et/ou de l'antigène DHFR et/ou de l'antigène CAT-GFP, ledit procédé comprenant l'étape suivante :
- détermination de la présence d'au moins un antigène dans un échantillon biologique préalablement prélevé d'un mammifère par formation *in vitro* d'un complexe immun, ledit complexe immun étant constitué de la protéine NcMIC1 liée à l'anticorps anti-NcMIC1 ou de la protéine NcMIC3 liée à l'anticorps anti-NcMIC3 ou la protéine DHFR liée à l'anticorps anti-DHFR ou la protéine CAT-GFP liée à l'anticorps anti-CAT-GFP, par comparaison avec un échantillon biologique référence.

La présente description divulgue un procédé de mise en évidence des antigènes NcMIC1 et/ou NcMIC3 et/ou DHFR et/ou CAT-GFP et des anticorps anti-NcMIC1 et/ou anti-NcMIC3 et/ou anti-DHFR et/ou anti-CAT-GFP.

Par « détection de l'agent infectieux avec des technologies d'immunologie » on désigne l'ensemble des techniques permettant de détecter des protéines antigéniques spécifiques des souches sauvages de *N. caninum* (*i.e.* protéines NcMIC1 et NcMIC3) et des protéines spécifiques des souches mutantes de l'invention (*i.e.* protéines DHFR et/ou CAT-GFP).

La détection des protéines antigéniques peut résulter d'expériences d'immunohistochimie, d'immunotransfert, de méthode immuno-enzymatique avec capture d'antigène (ELISA), d'immunochromatographie ou de protéomique bien connues de l'homme de l'art. Ces essais peuvent être réalisés à partir de différents échantillons biologiques.

Par « Immunohistochimie », on entend la détection d'antigènes dans des tissus fixés en utilisant des anticorps dirigés spécifiquement contre l'antigène et marqués. L'immunohistochimie avec des anticorps spécifiques des protéines DHFR et/ou CAT-GFP permettra de mettre en évidence l'inoculation des souches mutantes de l'invention à l'animal. L'immunohistochimie avec des anticorps spécifiques des protéines NcMIC1 et NcMIC3 permettra de mettre en évidence la contamination de l'animal par une souche sauvage de *N. caninum.*

Par « Immunotransfert », on entend la détection d'antigènes dans des échantillons biologiques après séparation des protéines de l'échantillon par gel d'électrophorèse et révélation avec des anticorps dirigés spécifiquement contre l'antigène et marqués. L'immunotransfert avec des anticorps spécifiques des protéines DHFR et/ou CAT-GFP permettra de mettre en évidence l'inoculation des souches mutantes à l'animal. L'immunotransfert avec des anticorps spécifiques des protéines NcMIC1 et NcMIC3 permettra de mettre en évidence la contamination de l'animal par une souche sauvage.

Par « Méthode immuno-enzymatique avec capture d'antigène (ELISA) » on désigne la détection d'antigènes par utilisation d'anticorps de capture dirigés spécifiquement contre l'antigène et fixé sur une plaque solide (ELISA indirect de type sandwich). L'antigène présent dans l'échantillon est capturé par l'anticorps spécifique puis sa présence est révélée par un deuxième anticorps marqué. L'ELISA avec des anticorps spécifiques des protéines DHFR et/ou CAT-GFP permettra de mettre en évidence l'inoculation des souches mutantes de l'invention à l'animal. L'ELISA avec des anticorps spécifiques des protéines NcMIC1 et NcMIC3 permettra de mettre en évidence la contamination de l'animal par une souche sauvage de *N. caninum.*

Par « Immunochromatographie » on désigne une méthode pour la détection d'antigènes basée sur la purification de l'échantillon par chromatographie d'affinité utilisant un anticorps spécifique de l'antigène marqué et fixé sur une colonne de chromatographie. L'immunochromatographie avec des anticorps spécifiques des protéines DHFR et/ou CAT-GFP permettra de mettre en évidence l'inoculation des souches mutantes de l'invention à l'animal. L'immunochromatographie avec des anticorps spécifiques des protéines NcMIC1 et NcMIC3 permettra de mettre en évidence la contamination de l'animal par une souche sauvage de *N. caninum.*

Par « détection de l'agent infectieux avec des technologies moléculaires » on désigne les techniques de biologie moléculaire bien connues de l'homme de l'art pour identifier la présence de séquences nucléotidiques spécifiques des souches sauvages et des souches mutantes et notamment par réaction d'amplification en chaîne par polymérase (PCR), PCR en temps réel, par diagnostic par polymorphisme de longueur des fragments de restriction (RFLP) pouvant être lié aux méthodes PCR ou par diagnostic à l'aide de sondes nucléiques.

La description divulgue un oligonucléotide consistant en une séquence d'acide nucléique choisie dans le groupe comprenant SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49 ou leurs séquences complémentaires.

| **Nom de l'amorce** | **Séquence 5' → 3'** | **n° de séquence** |
|---|---|---|
| ATG Ncmic1 | ATGGGCCAGTCGGTGGTTTT | SEQ ID NO: 35 |
| ATG Ncmic3 | ATGCGTGGCGGGGCGTCCGC | SEQ ID NO: 36 |
| ATG DHFR | ATGCAGAAACCGGTGTGTC | SEQ ID NO: 37 |
| ATG CATGFP | ATGCATGAGAAAAAAATCACTG | SEQ ID NO: 38 |
| stop Ncmic1 | TTACAATTCAGATTCACCCG | SEQ ID NO :39 |
| stop Ncmic3 | TTATCGAGCCGTTCCGCATTTG | SEQ ID NO :40 |
| stop DHFR | CTAGACAGCCATCTCCATCTG | SEQ ID NO :41 |
| stop CATGFP | TTAATCGAGCGGGTCCTGGT | SEQ ID NO :42 |
| Olig 1 | CAGATGGAGATGGCTGTCTAG | SEQ ID NO :43 |
| Olig 2 | CGCTTTCGTTCTGATTGACA | SEQ ID NO :44 |
| Olig 3 | AAAACCACCGACTGGCCCAT | SEQ ID NO :45 |
| Olig 4 | TCCTCTCGTTGTTGGAAGCT | SEQ ID NO :46 |
| Olig 5 | TAGCACGGGAAAGGATTGAC | SEQ ID NO :47 |
| Olig 6 | CAAGATCCGCCACAACATC | SEQ ID NO :48 |
| ORF CATGFP F3 | TTCATCATGCCGTTTGTGAT | SEQ ID NO: 49 |

Par « oligonucléotide », on désigne une séquence d'acide nucléique qui peut être utilisée comme amorce dans un procédé d'amplification ou comme sonde dans un procédé de détection. Dans la présente invention, les oligonucléotides consistent en une séquence d'au moins 15, préférentiellement 20 nucléotides, et préférentiellement moins de 30 nucléotides, capable de s'hybrider à une molécule d'ADN génomique ou à un ADN complémentaire. Par « hybridation », on désigne l'interaction physique existante entre deux molécules d'acide nucléique. Cette hybridation peut impliquer des homoduplex ADN/ADN ou ARN/ARN ou des hétéroduplex ADN/ARN.

Par « acide nucléique », on désigne une succession de nucléotides liés entre eux par des liaisons phosphodiester. Une molécule d'acide nucléique peut être linéaire, circulaire, simple brin, double brin, partiellement double brin. Les séquences d'acides nucléiques sont décrites dans la présente invention selon l'usage bien connu de l'homme de l'art, c'est-à-dire qu'elles sont définies par une séquence numérotée selon la direction 5' vers 3'.

Par « séquences complémentaires », on désigne deux séquences nucléiques qui possèdent des nucléotides complémentaires pouvant interagir entre eux par des liaisons hydrogènes. En face d'une adénine, il y a toujours une thymine ou un uracile (dans le cas d'un hétéroduplex ADN/ARN); en face d'une cytosine, il y a toujours une guanine. A titre d'exemple, et sans que cela ne restreigne la portée de l'invention, la séquence 5' ATCG 3' et la séquence 5' CGAT 3' sont complémentaires.

La description divulgue également les couples d'oligonucléotides consistant en les couples de séquences choisis parmi :
- SEQ ID NO: 21 et SEQ ID NO: 25, SEQ ID NO: 21 et SEQ ID NO: 28, SEQ ID NO: 21 et SEQ ID NO: 35, SEQ ID NO: 21 et SEQ ID NO: 46,
- SEQ ID NO: 39 et SEQ ID NO: 25, SEQ ID NO: 39 et SEQ ID NO: 28, SEQ ID NO: 39 et SEQ ID NO: 35, SEQ ID NO: 39 et SEQ ID NO: 46, SEQ ID NO: 39 et SEQ ID NO: 24,
- SEQ ID NO: 27 et SEQ ID NO: 28, SEQ ID NO: 27 et SEQ ID NO: 35, SEQ ID NO: 27 et SEQ ID NO: 46, SEQ ID NO: 27 et SEQ ID NO: 24,
- SEQ ID NO: 45 et SEQ ID NO: 46, SEQ ID NO: 45 et SEQ ID NO: 24,
- SEQ ID NO: 47 et SEQ ID NO: 46, SEQ ID NO: 47 et SEQ ID NO: 24,
- SEQ ID NO: 26 et SEQ ID NO: 24,
- SEQ ID NO: 11 et SEQ ID NO: 12, SEQ ID NO: 11 et SEQ ID NO: 8, SEQ ID NO: 11 et SEQ ID NO: 40, SEQ ID NO: 11 et SEQ ID NO: 6,
- SEQ ID NO: 5 et SEQ ID NO: 12, SEQ ID NO: 5 et SEQ ID NO: 8, SEQ ID NO: 5 et SEQ ID NO: 40, SEQ ID NO: 5 et SEQ ID NO: 6, SEQ ID NO: 5 et SEQ ID NO: 14,
- SEQ ID NO: 7 et SEQ ID NO: 12, SEQ ID NO: 7 et SEQ ID NO: 8, SEQ ID NO: 7 et SEQ ID NO: 40, SEQ ID NO: 7 et SEQ ID NO: 6, SEQ ID NO: 7 et SEQ ID NO: 14,
- SEQ ID NO: 36 et SEQ ID NO: 8, SEQ ID NO: 36 et SEQ ID NO: 40, SEQ ID NO: 36 et SEQ ID NO: 6, SEQ ID NO: 36 et SEQ ID NO: 14, SEQ ID NO: 36 et SEQ ID NO: 12,
- SEQ ID NO: 15 et SEQ ID NO: 6, SEQ ID NO: 15 et SEQ ID NO: 14,
- SEQ ID NO: 11 et SEQ ID NO: 13, SEQ ID NO: 11 et SEQ ID NO: 10, SEQ ID NO: 11 et SEQ ID NO: 41, SEQ ID NO: 11 et SEQ ID NO: 44, SEQ ID NO: 11 et SEQ ID NO: 6,
- SEQ ID NO: 5 et SEQ ID NO: 13, SEQ ID NO: 5 et SEQ ID NO: 10, SEQ ID NO: 5 et SEQ ID NO: 41, SEQ ID NO: 5 et SEQ ID NO: 44, SEQ ID NO: 5 et SEQ ID NO: 6, SEQ ID NO: 5 et SEQ ID NO: 14,
- SEQ ID NO: 37 et SEQ ID NO: 10, SEQ ID NO: 37 et SEQ ID NO: 41, SEQ ID NO: 37 et SEQ ID NO: 44, SEQ ID NO: 37 et SEQ ID NO: 6, SEQ ID NO: 37 et SEQ ID NO: 14,
- SEQ ID NO: 9 et SEQ ID NO: 10, SEQ ID NO: 9 et SEQ ID NO: 41, SEQ ID NO: 9 et SEQ ID NO: 44, SEQ ID NO: 9 et SEQ ID NO: 6, SEQ ID NO: 9 et SEQ ID NO: 14,
- SEQ ID NO: 43 et SEQ ID NO: 44, SEQ ID NO: 43 et SEQ ID NO: 6, SEQ ID NO: 43 et SEQ ID NO: 14,
- SEQ ID NO: 16 et SEQ ID NO: 44, SEQ ID NO: 16 et SEQ ID NO: 6, SEQ ID NO: 16 et SEQ ID NO: 14,
- SEQ ID NO: 21 et SEQ ID NO: 22, SEQ ID NO: 21 et SEQ ID NO: 30, SEQ ID NO: 21 et SEQ ID NO: 42,
- SEQ ID NO: 38 et SEQ ID NO: 30, SEQ ID NO: 38 et SEQ ID NO: 42, SEQ ID NO: 38 et SEQ ID NO: 24,
- SEQ ID NO: 49 et SEQ ID NO: 30, SEQ ID NO: 49 et SEQ ID NO: 42, SEQ ID NO: 49 et SEQ ID NO: 24,
- SEQ ID NO: 29 et SEQ ID NO: 30, SEQ ID NO: 29 et SEQ ID NO: 42, SEQ ID NO: 29 et SEQ ID NO: 24,
- SEQ ID NO: 23 et SEQ ID NO: 30, SEQ ID NO: 23 et SEQ ID NO: 42, SEQ ID NO: 23 et SEQ ID NO: 24,
- SEQ ID NO: 48 et SEQ ID NO: 30, SEQ ID NO: 48 et SEQ ID NO: 42, SEQ ID NO: 48 et SEQ ID NO: 24,
ou leurs séquences complémentaires.

Par « couple d'oligonucléotides », on désigne deux nucléotides tels que définis par leurs séquences.

Un autre but de la description est de proposer des sets d'oligonucléotides consistant en les triades de séquences choisis parmi :
- SEQ ID NO: 7 et SEQ ID NO: 12 et SEQ ID NO: 36,
- SEQ ID NO: 43 et SEQ ID NO: 44 et SEQ ID NO: 16,
- SEQ ID NO: 45 et SEQ ID NO: 46 et SEQ ID NO: 47,
- SEQ ID NO: 23 et SEQ ID NO: 30 et SEQ ID NO: 48,
ou leurs séquences complémentaires.

Pour chaque triade, les 2 premières SEQ ID correspondent aux amorces et la troisième correspond à la séquence de la sonde.

Par « sets d'oligonucléotides », on entend des groupes de trois oligonucléotides tels que définis par leurs séquences respectives.

Les souches sauvages de *Neospora caninum* possèdent dans leur génome les gènes *ncmic1* et *ncmic3.*

L'analyse, dans un échantillon biologique de la présence et/ou du niveau d'expression des quatre gènes *ncmic1, ncmic3, dhfr, cat-gfp* permet de déterminer si l'animal est porteur d'une souche de *Neospora caninum* résultant d'une infection par une souche sauvage ou bien résultant d'une vaccination par l'une des trois souches mutantes telles que décrites dans la présente invention. Le but est de pouvoir établir un diagnostic différentiel permettant de discriminer, au sein d'un troupeau, les animaux vaccinés des animaux non vaccinés et/ou infectés.

La description divulgue également l'utilisation d'au moins un oligonucléotide consistant en une séquence d'acide nucléique choisie dans le groupe comprenant SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49 ou leurs séquences complémentaires, pour la détection de gènes *ncmic1,* et/ou *ncmic3,* et/ou *dhfr,* et/ou *cat-gfp* provenant du génome de souches sauvages et/ou des souches mutantes Neo *ncmic1* KO et/ou Neo *ncmic3* KO et/ou Neo *ncmic1-3* KO de *Neospora caninum.*

La description divulgue l'utilisation d'au moins un oligonucléotide consistant en la séquence choisie parmi SEQ ID NO: 21, SEQ ID NO: 24, SEQ ID NO: 25, SEQ ID NO: 26, SEQ ID NO: 27, SEQ ID NO: 28, SEQ ID NO: 35, SEQ ID NO: 39, SEQ ID NO: 45, SEQ ID NO: 46, SEQ ID NO: 47 ou leur séquence complémentaire, comme amorce pour réaliser une hybridation et éventuellement une amplification du gène *ncmic1* provenant du génome de souches sauvages et/ou de la souche mutante Neo *ncmic3* KO de *Neospora caninum.*

La description divulgue l'utilisation d'oligonucléotides consistant en au moins un couple de séquences choisi parmi : SEQ ID NO: 21 et SEQ ID NO: 25, SEQ ID NO: 21 et SEQ ID NO: 28, SEQ ID NO: 21 et SEQ ID NO: 35, SEQ ID NO: 21 et SEQ ID NO: 46, SEQ ID NO: 39 et SEQ ID NO: 25, SEQ ID NO: 39 et SEQ ID NO: 28, SEQ ID NO: 39 et SEQ ID NO: 35, SEQ ID NO: 39 et SEQ ID NO: 46, SEQ ID NO: 39 et SEQ ID NO: 24, SEQ ID NO: 27 et SEQ ID NO: 28, SEQ ID NO: 27 et SEQ ID NO: 35, SEQ ID NO: 27 et SEQ ID NO: 46, SEQ ID NO: 27 et SEQ ID NO: 24, SEQ ID NO: 45 et SEQ ID NO: 46, SEQ ID NO: 45 et SEQ ID NO: 24, SEQ ID NO: 47 et SEQ ID NO: 46, SEQ ID NO: 47 et SEQ ID NO: 24, SEQ ID NO: 26 et SEQ ID NO: 24, ou leurs séquences complémentaires, comme amorces pour réaliser une hybridation et éventuellement une amplification du gène *ncmic1* provenant du génome de souches sauvages et/ou de la souche mutante Neo *ncmic3* KO de *Neospora caninum.*

La description divulgue l'utilisation d'au moins un oligonucléotide consistant en la séquence choisie parmi SEQ ID NO: 5, SEQ ID NO: 6, SEQ ID NO: 7, SEQ ID NO: 8, SEQ ID NO: 11, SEQ ID NO: 12, SEQ ID NO: 14, SEQ ID NO: 15, SEQ ID NO: 36, SEQ ID NO: 40, ou leur séquence complémentaire, comme amorce pour réaliser une hybridation et éventuellement une amplification du gène *ncmic3* provenant du génome de souches sauvages et/ou de la souche mutante Neo *ncmic1* KO de *Neospora caninum.*

La description divulgue l'utilisation d'oligonucléotides consistant en au moins un couple de séquences choisi parmi SEQ ID NO: 11 et SEQ ID NO: 12, SEQ ID NO: 11 et SEQ ID NO: 8, SEQ ID NO: 11 et SEQ ID NO: 40, SEQ ID NO: 11 et SEQ ID NO: 6, SEQ ID NO: 5 et SEQ ID NO: 12, SEQ ID NO: 5 et SEQ ID NO: 8, SEQ ID NO: 5 et SEQ ID NO: 40, SEQ ID NO: 5 et SEQ ID NO: 6, SEQ ID NO: 5 et SEQ ID NO: 14, SEQ ID NO: 7 et SEQ ID NO: 12, SEQ ID NO: 7 et SEQ ID NO: 8, SEQ ID NO: 7 et SEQ ID NO: 40, SEQ ID NO: 7 et SEQ ID NO: 6, SEQ ID NO: 7 et SEQ ID NO: 14, SEQ ID NO: 36 et SEQ ID NO: 8, SEQ ID NO: 36 et SEQ ID NO: 40, SEQ ID NO: 36 et SEQ ID NO: 6, SEQ ID NO: 36 et SEQ ID NO: 14, SEQ ID NO: 36 et SEQ ID NO: 12, SEQ ID NO: 15 et SEQ ID NO: 6, SEQ ID NO: 15 et SEQ ID NO: 14, ou leurs séquences complémentaires, comme amorces pour réaliser une hybridation et éventuellement une amplification du gène *ncmic3* provenant du génome de souches sauvages et/ou de la souche mutante Neo *ncmic1* KO de *Neospora caninum.*

La description divulgue l'utilisation d'au moins un oligonucléotide consistant en la séquence choisie parmi SEQ ID NO: 5, SEQ ID NO: 6, SEQ ID NO: 9, SEQ ID NO: 10, SEQ ID NO: 11, SEQ ID NO: 13, SEQ ID NO: 14, SEQ ID NO: 16, SEQ ID NO: 37, SEQ ID NO: 41, SEQ ID NO: 43, SEQ ID NO: 44, ou leur séquence complémentaire, comme amorce pour réaliser une hybridation et éventuellement une amplification du gène de sélection *dhfr* provenant du génome des souches mutantes Neo *ncmic3* KO et/ou Neo *ncmic1-3* KO de *Neospora caninum.*

La description divulgue l'utilisation d'oligonucléotides consistant en au moins un couple de séquences choisi parmi: SEQ ID NO: 11 et SEQ ID NO: 13, SEQ ID NO: 11 et SEQ ID NO: 10, SEQ ID NO: 11 et SEQ ID NO: 41, SEQ ID NO: 11 et SEQ ID NO: 44, SEQ ID NO: 11 et SEQ ID NO: 6, SEQ ID NO: 5 et SEQ ID NO: 13, SEQ ID NO: 5 et SEQ ID NO: 10, SEQ ID NO: 5 et SEQ ID NO: 41, SEQ ID NO: 5 et SEQ ID NO: 44, SEQ ID NO: 5 et SEQ ID NO: 6, SEQ ID NO: 5 et SEQ ID NO: 14, SEQ ID NO: 37 et SEQ ID NO: 10, SEQ ID NO: 37 et SEQ ID NO: 41, SEQ ID NO: 37 et SEQ ID NO: 44, SEQ ID NO: 37 et SEQ ID NO: 6, SEQ ID NO: 37 et SEQ ID NO: 14, SEQ ID NO: 9 et SEQ ID NO: 10, SEQ ID NO: 9 et SEQ ID NO: 41, SEQ ID NO: 9 et SEQ ID NO: 44, SEQ ID NO: 9 et SEQ ID NO: 6, SEQ ID NO: 9 et SEQ ID NO: 14, SEQ ID NO: 43 et SEQ ID NO: 44, SEQ ID NO: 43 et SEQ ID NO: 6, SEQ ID NO: 43 et SEQ ID NO: 14, SEQ ID NO: 16 et SEQ ID NO: 44, SEQ ID NO: 16 et SEQ ID NO: 6, SEQ ID NO: 16 et SEQ ID NO: 14, ou leurs séquences complémentaires, comme amorces pour réaliser une hybridation et éventuellement une amplification du gène de sélection *dhfr* provenant du génome de souches mutantes Neo *ncmic3* KO et/ou Neo *ncmic1-3* KO *Neospora caninum.*

La description divulgue l'utilisation d'au moins un oligonucléotide consistant en la séquence choisie parmi SEQ ID NO: 21, SEQ ID NO: 22, SEQ ID NO: 23, SEQ ID NO: 24, SEQ ID NO: 29, SEQ ID NO: 30, SEQ ID NO: 38, SEQ ID NO: 42, SEQ ID NO: 48, SEQ ID NO: 49 ou leur séquence complémentaire, comme amorce pour réaliser une hybridation et éventuellement une amplification du gène de sélection *cat-gfp* provenant du génome des souches mutantes Neo *ncmic1* KO et/ou Neo *ncmic1-3* KO de *Neospora caninum.*

La description divulgue l'utilisation d'oligonucléotides consistant en au moins un couple de séquences choisi parmi: SEQ ID NO: 21 et SEQ ID NO: 22, SEQ ID NO: 21 et SEQ ID NO: 30, SEQ ID NO: 21 et SEQ ID NO: 42, SEQ ID NO: 38 et SEQ ID NO: 30, SEQ ID NO: 38 et SEQ ID NO: 42, SEQ ID NO: 38 et SEQ ID NO: 24, SEQ ID NO: 49 et SEQ ID NO: 30, SEQ ID NO: 49 et SEQ ID NO: 42, SEQ ID NO: 49 et SEQ ID NO: 24,SEQ ID NO: 29 et SEQ ID NO: 30, SEQ ID NO: 29 et SEQ ID NO: 42, SEQ ID NO: 29 et SEQ ID NO: 24, SEQ ID NO: 23 et SEQ ID NO: 30, SEQ ID NO: 23 et SEQ ID NO: 42, SEQ ID NO: 23 et SEQ ID NO: 24, SEQ ID NO: 48 et SEQ ID NO: 30, SEQ ID NO: 48 et SEQ ID NO: 42, SEQ ID NO: 48 et SEQ ID NO: 24, ou leurs séquences complémentaires, comme amorces pour réaliser une hybridation et éventuellement une amplification du gène de sélection *cat-gfp* provenant du génome de souches mutantes Neo *ncmic1* KO et/ou Neo *ncmic1-3* KO *Neospora caninum.*

Par « amplification », on désigne l'augmentation de la concentration d'une séquence d'ADN spécifique parmi un mélange de séquences d'ADN. Les techniques d'amplification de l'ADN sont des techniques bien connues de l'homme de l'art.

La description divulgue également l'utilisation d'au moins un oligonucléotide consistant en une séquence d'acide nucléique choisie dans le groupe comprenant SEQ ID NO: 35 à 42, SEQ ID NO: 16, SEQ ID NO: 47, SEQ ID NO: 48, ou de sa séquence complémentaire, comme sonde pour réaliser une hybridation avec un acide nucléique provenant du génome de souches sauvages et/ou des souches mutantes Neo *ncmic1* KO et/ou Neo *ncmic3* KO et/ou Neo *ncmic1-3* KO de *Neospora caninum.*

La description divulgue également l'utilisation de l'oligonucléotide consistant en une séquence d'acide nucléique choisie dans le groupe comprenant SEQ ID NO: 35 à 42, SEQ ID NO: 16, SEQ ID NO: 47, SEQ ID NO: 48, ou de sa séquence complémentaire, le susdit oligonucléotide étant marqué à une extrémité par un fluorophore et éventuellement à l'autre extrémité par un quencher.

Par « fluorophore », on désigne les molécules capables d'émettre de la lumière lorsqu'elles sont excitées par une source lumineuse. Les fluorophores sont des molécules bien connues par l'homme de l'art, dont les plus utilisés sont Fam, Tet, Hex, Tamra, Texas Red, Cy3, Cy5. Cette liste non exhaustive a pour but d'illustrer la notion de fluorophore mais ne doit en aucun cas restreindre la présente invention à l'utilisation de ces seuls fluorophores.

Par « quencher », on désigne une espèce chimique, capable de désactiver un état excité créé dans une entité moléculaire par transfert d'énergie, d'électron ou par un mécanisme chimique. Les quenchers sont des molécules bien connues par l'homme de l'art, dont les plus utilisés sont Dabcyl, Eclipse Dark Quencher, Black Hole Quencher. Un fluorophore peut également servir de quencher. Pour cela, le spectre d'émission du fluorophore greffé en 5' ne doit pas chevaucher le spectre d'excitation du fluorophore-quencher greffé en 3'. Cette liste non exhaustive a pour but d'illustrer la notion de quencher mais ne doit en aucun cas restreindre la présente invention à l'utilisation de ces seuls quenchers.

Dans la présente invention, les sondes utilisées peuvent rentrer dans la définition de la technologie Taqman, FRET (Fluorescent Resonnance Energy Transfer), Molecular Beacon ou Scorpion ou toute autre technologie de PCR (ou RT-PCR) en temps réel bien connue de l'homme de l'art.

La description divulgue également un procédé de détection de *Neospora caninum* par amplification *in vitro* à partir d'un échantillon biologique, ledit procédé comportant les étapes de :
- mise en contact du set d'oligonucléotides tel que défini ci-dessus avec un échantillon biologique, ou un acide nucléique issu du susdit échantillon biologique, dans des conditions permettant aux oligonucléotides de s'hybrider à un acide nucléique de *Neospora caninum* présent dans le susdit échantillon,
- amplification de l'acide nucléique de *Neospora caninum* en utilisant les oligonucléotides comme amorces,
- détection du produit d'amplification caractérisant la présence de *Neospora caninum* dans l'échantillon.

Le procédé de détection peut être mis en oeuvre sur un échantillon biologique choisi parmi le groupe constitué par le sang, le sérum ou le plasma mais également certains tissus et organes tels que le placenta, le cerveau, les muscles, *etc.*

Dans le procédé de détection de *Neospora caninum*, l'acide nucléique de *Neospora caninum* est amplifié par PCR. La PCR peut être qualitative, quantitative ou semi-quantitative. Suivant qu'une sonde de détection est utilisé ou pas, on parle de PCR en temps réel ou de PCR classique.

Dans le procédé de détection de *Neospora caninum*, la détection du produit d'amplification est réalisée en utilisant au moins l'un des oligonucléotides de séquence SEQ ID NO: 35 à 42, SEQ ID NO: 16, SEQ ID NO: 47, SEQ ID NO: 48, ou sa séquence complémentaire, ou de tout autre oligonucléotide de séquence incluse dans celle de l'amplicon obtenu à partir des amorces ayant permis l'amplification du fragment génique d'intérêt, marqué à une extrémité par un fluorophore et à l'autre extrémité par un quencher comme sonde.

La description divulgue également un kit d'amplification de *Neospora caninum* à partir d'un échantillon biologique, ledit kit comprenant l'un des susdits sets d'oligonucléotides, ou leurs séquences complémentaires, et des moyens permettant l'amplification d'un acide nucléique de *Neospora caninum.*

Selon un mode de réalisation particulier, ledit kit d'amplification comprend :
- au moins un set d'oligonucléotides consistant en les oligonucléotides de séquences
   - SEQ ID NO: 7 et SEQ ID NO: 12 et SEQ ID NO: 36,
   - SEQ ID NO: 43 et SEQ ID NO: 44 et SEQ ID NO: 16,
   - SEQ ID NO: 45 et SEQ ID NO: 46 et SEQ ID NO: 47,
   - SEQ ID NO: 23 et SEQ ID NO: 30 et SEQ ID NO: 48, et
- un moyen pour amplifier un acide nucléique de *Neospora caninum,*
- éventuellement un contrôle interne.

Par « moyen pour amplifier un acide nucléique », on désigne les dNTPs, une Taq Polymérase, les sels et tampons qui permettent la réalisation d'une PCR.

Par « contrôle interne », on désigne une séquence nucléique (ADN exogène) sans rapport avec le génome de *Neospora caninum,* des amorces et une sonde permettant l'amplification et la détection de cet ADN exogène. Ce contrôle interne est placé dans le mix servant à la PCR pour la détection de *Neospora caninum* et atteste du bon fonctionnement de l'amplification.

Les figures et les exemples suivants visent à illustrer davantage la description ou l'invention.

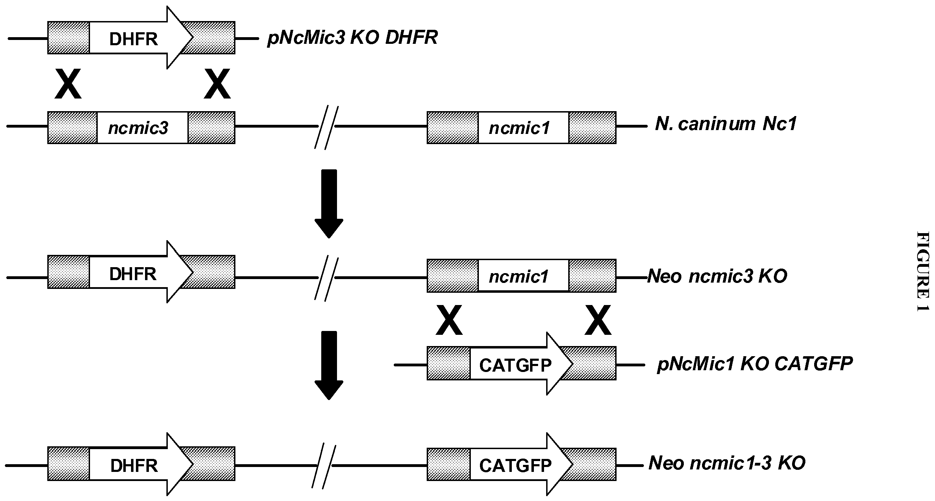

Figure 1 : cette figure illustre les 2 étapes de recombinaison homologue pour obtenir la souche Neo *ncmic1-3* KO. La première étape de recombinaison homologue permet l'intégration du gène codant pour l'enzyme dihydrofolate réductase (DHFR) au locus du gène *ncmic3.* Une sélection avec la pyriméthamine permet d'amplifier la souche simple mutante Neo *ncmic3* KO. La souche Neo *ncmic3* KO ainsi obtenue sert à la seconde étape de recombinaison homologue qui permet l'intégration du gène codant pour la protéine chimérique chloramphénicol-acétyl-transférase/green fluorescent protein (CAT-GFP) au locus du gène *ncmic1.* Une sélection avec le chloramphénicol permet ensuite d'amplifier la souche double mutante Neo *ncmic1-3* KO.

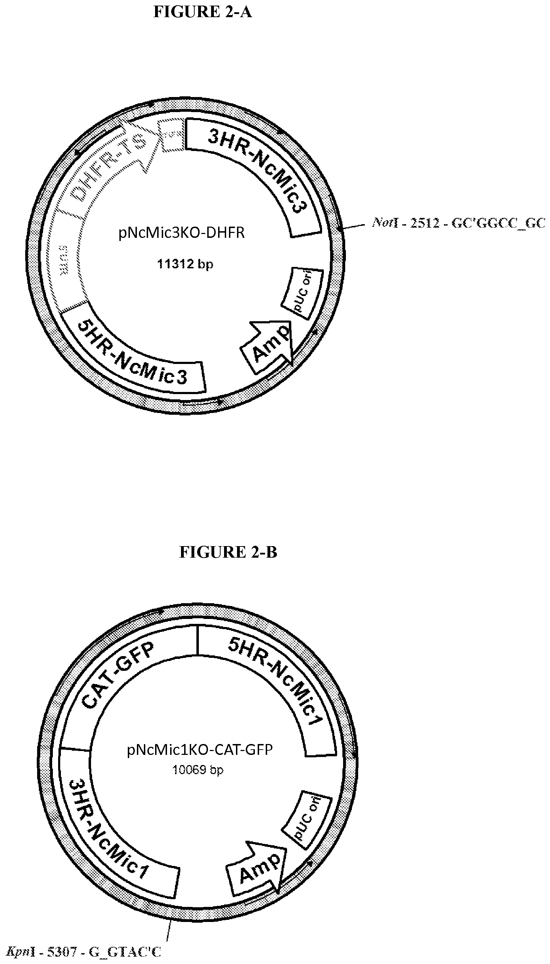

Figure 2-A : cette figure est une représentation schématique du plasmide pNcMic3KO-DHFR. Ce plasmide de 11 312 paires de bases comprend le gène de sélection *DHFR* flanqué par les régions homologues (5HR-NcMic3 et 3HR-NcMic3) des séquences flanquant le gène *ncmic3,* le gène de résistance à l'ampicilline (Amp) ainsi que le site de restriction Not I qui permet sa linéarisation.
Figure 2-B : cette figure est une représentation schématique du plasmide pNcMic1KO-CAT-GFP. Ce plasmide de 10 069 paires de bases comprend le gène de sélection CAT-GFP flanqué par les régions homologues (3HR-NcMicl et 5HR-NcMic1) des séquences flanquant le gène *ncmic1*, le gène de résistance à l'ampicilline (Amp) ainsi que le site de restriction Kpn I qui permet sa linéarisation.

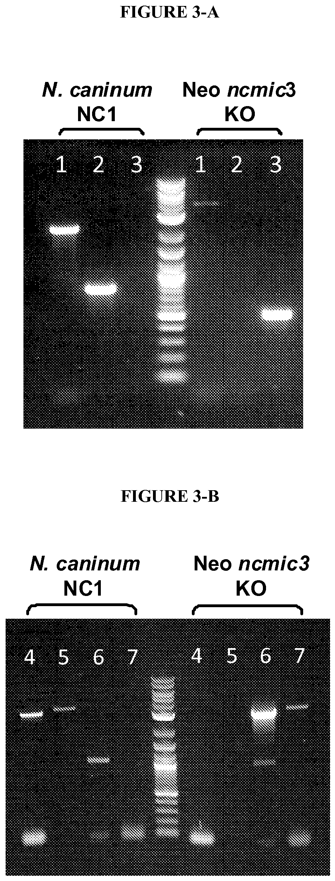

Figure 3-A : cette figure représente les profils électrophorétiques des produits PCR obtenus respectivement chez la souche sauvage NC1 de *N. caninum* et chez la souche mutante Neo *ncmic3* KO, en utilisant les jeux d'amorces des PCR n°1, n°2 ou n°3 du Tableau II qui correspondent aux SEQ ID NO : 5 à SEQ ID NO : 10.
Figure 3-B : cette figure représente les profils électrophorétiques des produits PCR obtenus respectivement chez la souche sauvage NC1 de *N. caninum* et chez la souche mutante Neo *ncmic3* KO, en utilisant les jeux d'amorces des PCR n°4, n°5, n°6 ou n°7 du Tableau II qui correspondent aux SEQ ID NO : 11 à SEQ ID NO : 16.

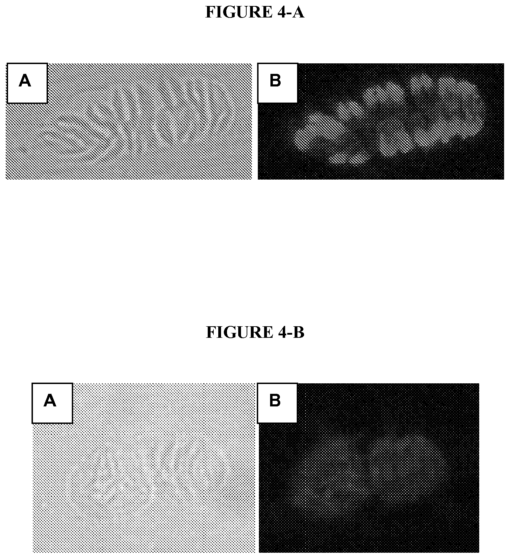

Figure 4-A : cette figure illustre l'analyse de la détection de la protéine NcMIC3 chez la souche sauvage NC1 de *N. caninum* par immunofluorescence, en utilisant un anticorps reconnaissant spécifiquement la protéine NcMIC3. Un même champ microscopique est visualisé en lumière directe (image A) ou en fluorescence (image B).
Figure 4-B : cette figure illustre l'analyse de la détection de la protéine NcMIC3 chez la souche mutante de *N. caninum* Neo *ncmic3* KO par immuno fluorescence, en utilisant un anticorps spécifiquement dirigé contre la protéine NcMIC3. Un même champ microscopique est visualisé en lumière directe (image A) ou en fluorescence (image B).

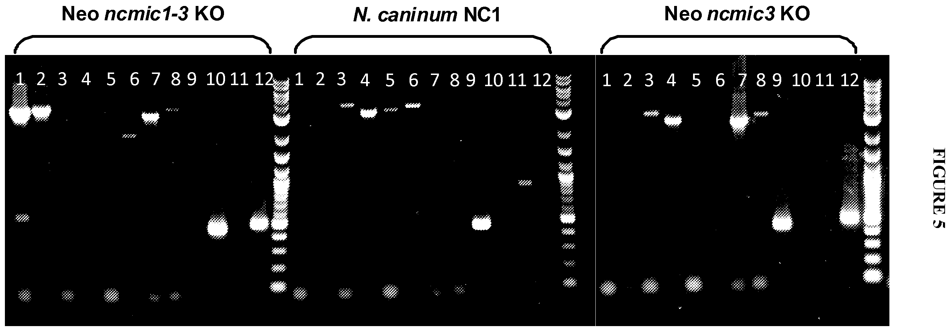

Figure 5 : cette figure représente les profils électrophorétiques des produits PCR obtenus respectivement chez la souche sauvage NC1 de *N. caninum*, chez la souche mutante Neo *ncmic3* KO et chez la souche mutante Neo *ncmic1-3* KO en utilisant les jeux d'amorces des PCR n°1 à n°12 du Tableau VII qui correspondent aux SEQ ID NO : 7 à 16 et aux SEQ ID NO : 21 à 30.

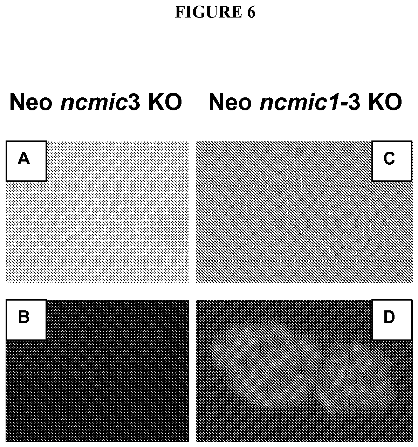

Figure 6 : cette figure illustre l'analyse de la détection de la protéine GFP chez les souches mutantes Neo *ncmic3* KO (images A et B) et Neo *ncmic1-3* KO (image C et D) par immunofluorescence, en utilisant les propriétés fluorescentes de la protéine CAT-GFP. Un même champ microscopique est visualisé en lumière directe (images hautes A et C) ou en fluorescence (images basses B et D).

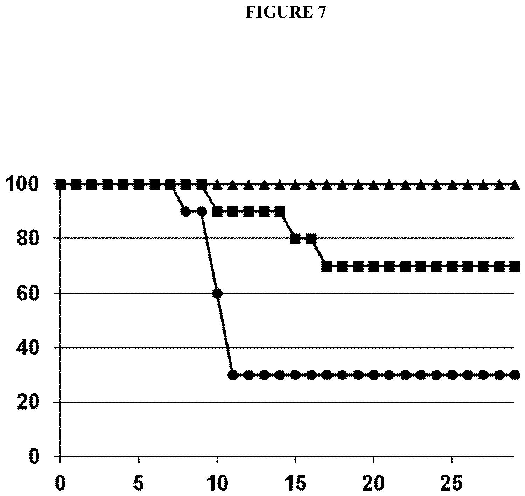

Figure 7 : cette figure représente le pourcentage de survie (en ordonnée) des souris femelles Balb/C infectées par voie intrapéritonéale avec 10⁷ tachyzoïtes de la souche sauvage NC1 de *Neospora caninum* (cercles noirs) ou des souches mutantes Neo *ncmic3* KO (carrés noirs) et Neo *ncmic1-3* KO (triangles noirs). L'axe des abscisses représente le temps écoulé après l'injection des tachyzoïtes aux souris (en jours).

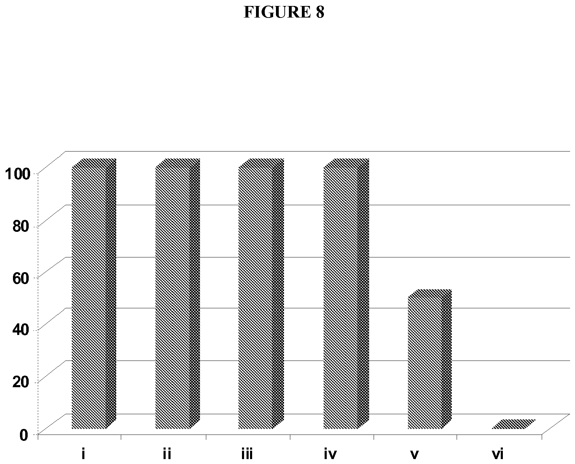

Figure 8 : cette figure représente le pourcentage de survie (en ordonnée) des souris femelles Balb/C après vaccination avec des doses croissantes de la souche mutante *ncmic1-3* KO et challenge 4 mois post-vaccination, avec une dose létale de la souche sauvage NC1 de *Neospora caninum.* Six lots de souris sont représentés sur l'axe des abscisses :
   Lot i : les souris femelles Balb/C de ce lot ont été vaccinées par voie intrapéritonéale avec 5x10⁶ tachyzoïtes de la souche mutante Neo *ncmic1-3* KO puis challengées par voie intrapéritonéale avec 2x10⁷ tachyzoïtes de la souche sauvage NC1 de *Neospora caninum.*
   Lot ii : les souris femelles Balb/C de ce lot ont été vaccinées par voie intrapéritonéale avec 10⁷ tachyzoïtes de la souche mutante Neo *ncmic1-3* KO puis challengées par voie intrapéritonéale avec 2x10⁷ tachyzoïtes de la souche sauvage NC1 de *Neospora caninum.*
   Lot iii : les souris femelles Balb/C de ce lot ont été vaccinées par voie intrapéritonéale avec une première dose de 10⁷ tachyzoïtes de la souche mutante Neo *ncmic1-3* KO, puis un mois plus tard une seconde dose de 10⁷ tachyzoïtes de la souche mutante Neo *ncmic1-3* KO, puis challengées par voie intrapéritonéale avec 2x10⁷ tachyzoïtes de la souche sauvage NC1 de *Neospora caninum.*
   Lot iv : les souris femelles Balb/C de ce lot ont été vaccinées par voie intrapéritonéale avec 5x10⁷ tachyzoïtes de la souche mutante Neo *ncmic1-3* KO puis challengées par voie intrapéritonéale avec 2x10⁷ tachyzoïtes de la souche sauvage NC1 de *Neospora caninum.*
   Lot v : les souris femelles Balb/C de ce lot ont été vaccinées par voie intrapéritonéale avec 10⁸ tachyzoïtes de la souche mutante Neo *ncmic1-3* KO puis challengées par voie intrapéritonéale avec 2x10⁷ tachyzoïtes de la souche sauvage NC1 de *Neospora caninum.*
   Lot vi : les souris femelles Balb/C de ce lot n'ont pas été vaccinées avec les tachyzoïtes de la souche mutante Neo *ncmic1-3* KO mais challengées par voie intrapéritonéale avec 2x10⁷ tachyzoïtes de la souche sauvage NC1 de *Neospora caninum.*

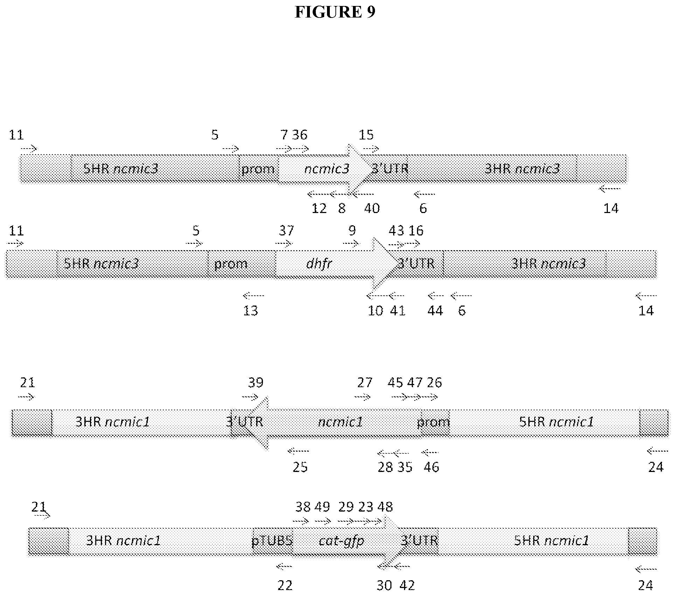

Figure 9 : cette figure illustre la position des amorces nucléiques utilisées dans la description ou l'invention pour détecter la présence des quatre gènes *ncmic3*, *dhfr*, *ncmic1*, *cat-gfp* dans les souches sauvages et/ou des souches mutantes de Neo *ncmic1* KO et/ou Neo *ncmic3* KO et/ou Neo *ncmic1-3* KO de *Neospora caninum.* Les chiffres représentent la numérotation des séquences d'amorces définies dans la présente description.

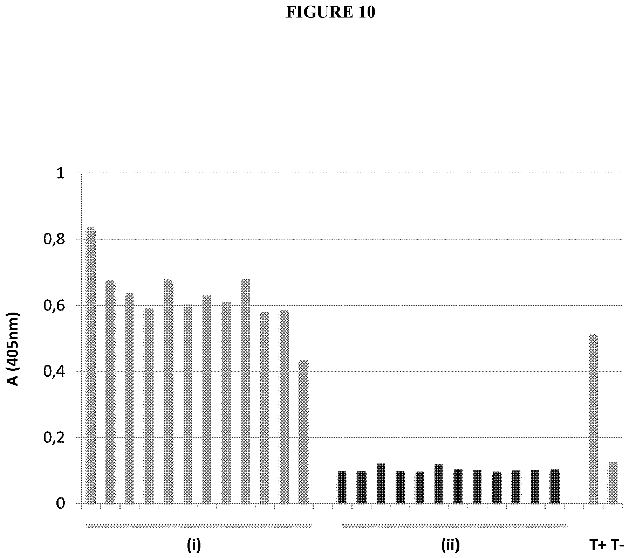

Figure 10 : cette figure représente les résultats des tests ELISA réalisés pour doser les anticorps IgG anti-*N*. *caninum* (densité optique à 405 nm en ordonnées), présents dans les sérums des souris vaccinées 30 jours au préalable par la souche Neo *ncmic1-3* KO ou non vaccinées. Quatre lots de souris sont représentés sur l'axe des abscisses :
   Lot i : les souris femelles de ce lot ont été vaccinées avec les tachyzoïtes de la souche mutante Neo *ncmic1-3* KO.
   Lot ii : les souris femelles de ce lot n'ont pas été vaccinées et sont donc naïves vis-à-vis de la néosporose.
   T+ : une souris infectée par *N. caninum* et présentant des anticorps IgG anti-*N*. *caninum* dans son sérum. Cette souris sert de témoin positif.
   T- : une souris naïve ne présentant pas d'anticorps IgG anti-*N*. *caninum* dans son sérum. Cette souris sert de témoin négatif.

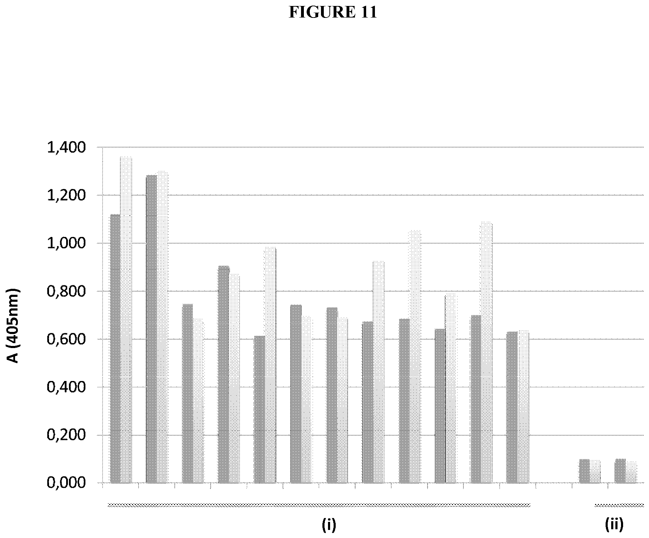

Figure 11 : cette figure représente les résultats des tests ELISA réalisés pour doser les anticorps IgG1 (histogramme gris foncé) et IgG2A (histogramme gris clair) anti-*N*. *caninum* (densité optique à 405 nm en ordonnées), présents dans les sérums des souris vaccinées 30 jours au préalable par la souche Neo *ncmic1-3* KO (12 souris incluses dans cette analyse) ou non (2 souris incluses dans cette analyse). Deux lots de souris sont représentés sur l'axe des abscisses :
   Lot i : les souris femelles de ce lot ont été vaccinées avec les tachyzoïtes de la souche mutante Neo *ncmic1-3* KO.
   Lot ii : les souris femelles de ce lot n'ont pas été vaccinées et sont donc naïves vis-à-vis de la néosporose.

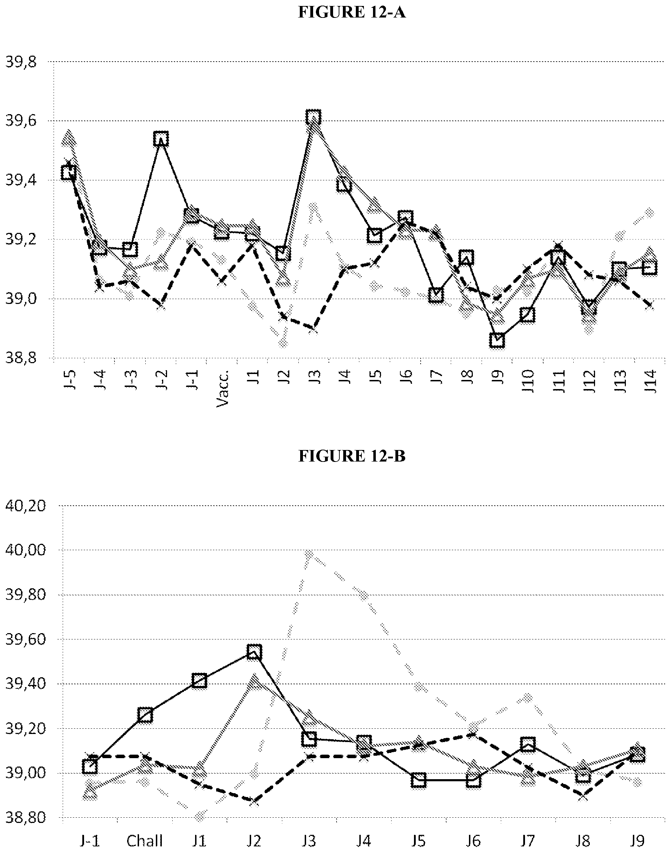

Figure 12-A : cette figure représente l'évolution de la température rectale moyenne en degré celcius (en ordonnée) des brebis de J-5 à J14 post-vaccination (en abscisse). Quatre lots sont représentés sur cette figure :
   Lot i : (courbe grise discontinue - rond gris) les brebis femelles de ce lot n'ont pas été vaccinées avec les tachyzoïtes de la souche mutante Neo *ncmic1-3* KO mais ont été fécondées, puis challengées à mi-gestation, par voie sous-cutanée avec 10⁷ tachyzoïtes de la souche sauvage NC1 de *Neospora caninum.*
   Lot ii (courbe noire continue - carré noir) : les brebis femelles de ce lot ont été vaccinées par voie sous-cutanée avec une première dose de 10⁷ tachyzoïtes de la souche mutante Neo *ncmic1-3* KO, puis un mois plus tard une seconde dose de 10⁷ tachyzoïtes de la souche mutante Neo *ncmic1-3* KO. Les brebis ont été fécondées 2 mois après la première vaccination, puis challengées, à mi-gestation, par voie sous-cutanée avec 10⁷ tachyzoïtes de la souche sauvage NC1 de *Neospora caninum.*
   Lot iii (courbe grise continue - triangle gris) : les brebis femelles de ce lot ont été vaccinées par voie sous-cutanée avec une dose de 10⁸ tachyzoïtes de la souche mutante Neo *ncmic1-3* KO. Les brebis ont été fécondées 2 mois après la vaccination, puis challengées, à mi-gestation, par voie sous-cutanée avec 10⁷ tachyzoïtes de la souche sauvage NC1 de *Neospora caninum.*
   Lot iv (courbe noire discontinue - croix noire) : les brebis femelles de ce lot n'ont été ni vaccinées avec les tachyzoïtes de la souche mutante Neo *ncmic1-3* KO ni challengées avec 10⁷ tachyzoïtes de la souche sauvage NC1 de *Neospora caninum.* Elles ont été fécondées en même temps que les brebis du lot (i), (ii) et (iii).
Figure 12-B : cette figure représente l'évolution de la température rectale moyenne en degré celcius (en ordonnée) des brebis de J-1 à J9 post-challenge (en abscisse). Quatre lots sont représentés sur cette figure :
   Lot i : (courbe grise discontinue - rond gris) les brebis femelles de ce lot n'ont pas été vaccinées avec les tachyzoïtes de la souche mutante Neo *ncmic1-3* KO mais ont été fécondées, puis challengées à mi-gestation, par voie sous-cutanée avec 10⁷ tachyzoïtes de la souche sauvage NC1 de *Neospora caninum.*
   Lot ii (courbe noire continue - carré noir) : les brebis femelles de ce lot ont été vaccinées par voie sous-cutanée avec une première dose de 10⁷ tachyzoïtes de la souche mutante Neo *ncmic1-3* KO, puis un mois plus tard une seconde dose de 10⁷ tachyzoïtes de la souche mutante Neo *ncmic1-3* KO. Les brebis ont été fécondées 2 mois après la première vaccination, puis challengées, à mi-gestation, par voie sous-cutanée avec 10⁷ tachyzoïtes de la souche sauvage NC1 de *Neospora caninum.*
   Lot iii (courbe grise continue - triangle gris) : les brebis femelles de ce lot ont été vaccinées par voie sous-cutanée avec une dose de 10⁸ tachyzoïtes de la souche mutante Neo *ncmic1-3* KO. Les brebis ont été fécondées 2 mois après la vaccination, puis challengées, à mi-gestation, par voie sous-cutanée avec 10⁷ tachyzoïtes de la souche sauvage NC1 de *Neospora caninum.*
   Lot iv (courbe noire discontinue - croix noire) : les brebis femelles de ce lot n'ont été ni vaccinées avec les tachyzoïtes de la souche mutante Neo *ncmic1-3* KO ni challengées avec 10⁷ tachyzoïtes de la souche sauvage NC1 de *Neospora caninum.* Elles ont été fécondées en même temps que les brebis du lot (i), (ii) et (iii).

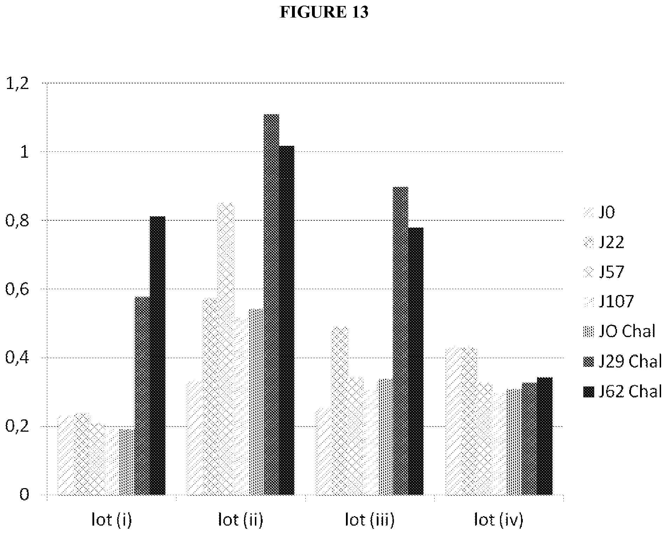

Figure 13 : cette figure représente les moyennes des résultats des tests ELISA (densité optique à 405 nm en ordonnées), réalisés à partir des sérums des brebis le jour de la vaccination du lot (ii) et (iii) (J0), le jour du boost du lot (ii) (J22), 57 jours après la première vaccination (J57), 107 jours après la première vaccination (J107), le jour du challenge (J0 Chal), 29 jours après challenge (J29 Chal) et 62 jours après challenge (J62 Chal). Quatre lots sont représentés en abscisse sur cette figure :
   Lot i : les brebis femelles de ce lot n'ont pas été vaccinées avec les tachyzoïtes de la souche mutante Neo *ncmic1-3* KO mais ont été fécondées, puis challengées à mi-gestation, par voie sous-cutanée avec 10⁷ tachyzoïtes de la souche sauvage NC1 de *Neospora caninum.*
   Lot ii : les brebis femelles de ce lot ont été vaccinées par voie sous-cutanée avec une première dose de 10⁷ tachyzoïtes de la souche mutante Neo *ncmic1-3* KO, puis un mois plus tard une seconde dose de 10⁷ tachyzoïtes de la souche mutante Neo *ncmic1-3* KO. Les brebis ont été fécondées 2 mois après la première vaccination, puis challengées, à mi-gestation, par voie sous-cutanée avec 10⁷ tachyzoïtes de la souche sauvage NC1 de *Neospora caninum.*
   Lot iii : les brebis femelles de ce lot ont été vaccinées par voie sous-cutanée avec une dose de 10⁸ tachyzoïtes de la souche mutante Neo *ncmic1-3* KO. Les brebis ont été fécondées 2 mois après la vaccination, puis challengées, à mi-gestation, par voie sous-cutanée avec 10⁷ tachyzoïtes de la souche sauvage NC1 de *Neospora caninum.*
   Lot iv : les brebis femelles de ce lot n'ont été ni vaccinées avec les tachyzoïtes de la souche mutante Neo *ncmic1-3* KO ni challengées avec 10⁷ tachyzoïtes de la souche sauvage NC1 de *Neospora caninum.* Elles ont été fécondées en même temps que les brebis du lot (i), (ii) et (iii).

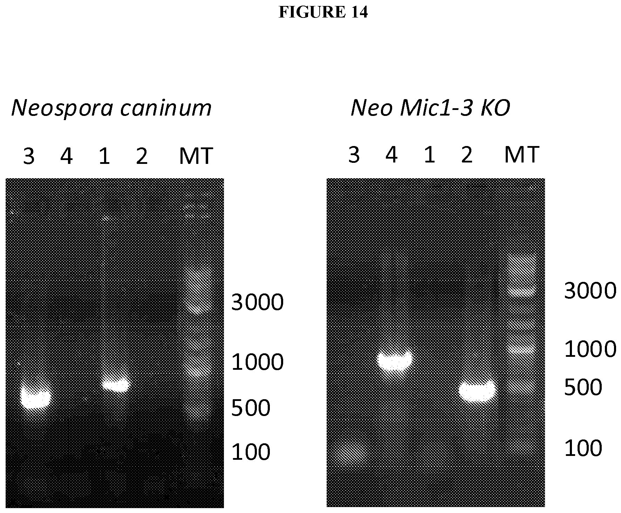

Figure 14 : cette figure représente les profils électrophorétiques des produits PCR obtenus respectivement à partir de cerveaux de souris infectées par *Neospora caninum* ou à partir de cerveaux de souris vaccinées avec la souche Neo *ncmic3* KO, en utilisant les jeux d'amorces des PCR n°1 (SEQ ID NO: 7 et SEQ ID NO: 8), n°2 (SEQ ID NO: 9 et SEQ ID NO: 10), n°3 (SEQ ID NO: 39 et SEQ ID NO: 25) ou n°4 (SEQ ID NO: 49 et SEQ ID NO: 42) définies dans le Tableau XVI.

### Exemples

Afin d'élaborer la souche de *N. caninum* invalidée pour les gènes *ncmic1* et *ncmic3*, deux étapes de recombinaison homologue ont été effectuées. La première étape de recombinaison homologue permet l'obtention d'un simple mutant KO (souche Neo *ncmic3* KO). La deuxième étape de recombinaison homologue se fait dans la souche Neo *ncmic3* KO pour obtenir une souche doublement délétée (Neo *ncmic1-3* KO) (Figure 1).

### Exemple 1 : Construction de la souche mutante Neo ncmic3 KO

L'haploïdie du génome de *Neospora caninum* lors de la phase proliférative permet l'invalidation d'un gène en une seule recombinaison homologue.

Tous les tachyzoïtes de la souche NC1 de *Neospora caninum* utilisés ont été produits en fibroblastes humains (HFF) cultivés en milieu minimal de Dulbecco (DMEM) supplémenté par 10% de sérum de veau foetal (FCS), 2 mM glutamine, 50 U/mL de pénicilline et 50 µg de streptomycine. Ils ont été récoltés après lyse mécanique des cellules hôtes et 3 passages en seringue 25G.

### a) Construction du plasmide pNcMic3KO-DHFR

Le plasmide **pNcMic3KO-DHFR** (Figure 2-A) contient le gène de sélection *DHFR* (dihydrofolate reductase) conférant une résistance à la pyriméthamine (Donald et al., PNAS, 1993, 90(24):11703-11707). Le gène de sélection *DHFR* est placé sous le contrôle du promoteur *tgdhfr* de *Toxoplasma gondii* (promoteur *tgdhfr)* pour permettre l'expression du gène dans le parasite. L'efficacité de ce promoteur hétérologue a été démontrée préalablement chez *N. caninum.* Cette cassette est encadrée par les régions homologues (5HR-NcMic3 et 3HR-NcMic3) des séquences flanquant le gène *ncmic3.* La cassette de sélection DHFR permet d'effectuer une sélection à la pyriméthamine.

La région 5'UTR du gène *ncmic3* a été amplifiée par PCR à partir de l'ADN génomique de la souche NC1 de *Neospora caninum.* Pour l'amplification, les amorces 5 HR NCmic3 F KpnI et 5 HR NCmic3 R ClaI (SEQ ID NO : 1 et SEQ ID NO : 2) permettent l'amplification de la région 5'UTR du gène *ncmic3* et la création de deux sites de restriction qui ont été utilisés pour cloner le fragment 5HR en amont de la cassette de sélection DHFR dans le plasmide pT230 DHFR (KpnI en 5' et ClaI en 3' du fragment de PCR).

La région 3'UTR du gène *ncmic3* a été amplifiée par PCR à partir de l'ADN génomique de la souche NC1 de *Neospora caninum.* Pour l'amplification, les amorces 3 HR NCmic3 F Xbal et 3 HR NCmic3 R NotI (SEQ ID NO : 3 et SEQ ID NO : 4) permettent l'amplification de la région 3'UTR du gène *ncmic3* et la création de deux sites de restriction qui ont été utilisés pour cloner le fragment 3HR en aval de la cassette de sélection DHFR dans le plasmide pT230 5HR-NcMic3-DHFR (XbaI en 5' et NotI en 3' du fragment de PCR). Les séquences des amorces figurent dans le Tableau I ci-dessous.

**Tableau I : Liste des amorces servant à l'intégration des séquences 5'UTR et 3'UTR du gène ncmic3. Les séquences des sites de restrictions sont soulignées.**

| **Nom de l'amorce** | **Séquence 5' → 3'** | **n° de séquence** |
|---|---|---|
| 5 HR NCmic3 F KpnI | CGCGGTACCCATGTGAATATGCTTTAACCGTGAC | SEQ ID NO: 1 |
| 5 HR NCmic3 R ClaI | CGCATCGATGAGCTATAACCCTTGGAAATGACTC | SEQ ID NO: 2 |
| 3 HR NCmic3 F XbaI | CGCTCTAGACATGCTGATGAAGAAGGGAAGT | SEQ ID NO: 3 |
| 3 HR NCmic3 R NotI | CGCGCGGCCGCTCTCTCCTGAAGTCTTCGAGACC | SEQ ID NO: 4 |

### b) Conditions d'électroporation et de sélection

50 µg du plasmide pNcMic3KO-DHFR purifié puis linéarisé par *NotI* ont été ajoutés à 5x10⁷ tachyzoïtes NC1 de *Neospora caninum* mis en suspension dans le milieu d'électroporation CYTOMIX contenant de l'ATP (3 mM) et du Glutathion (3 mM) (Van den Hoff et al., Nucleic Acid Research, Jun 11; 20(11):2902), et l'électroporation a été réalisée en cuvette d'écart 4 mm, dans un volume de 800 µL sur un appareil BioRad (Paramètres : 2000 V, 50 ohms, 25 µF, avec deux chocs électriques).

Après électroporation, les tachyzoïtes ont été déposés sur une monocouche de cellules HFF en culture. Pour la sélection des mutants, le milieu de culture est remplacé et supplémenté par l'agent de sélection (pyriméthamine 2 µM), 24h après l'électroporation. Trois passages en culture sont effectués dans ce milieu.

Après 16 jours de sélection, les parasites résistants sont clonés par dilution limite dans les puits de plaques de 96 puits de cellules HFF. Après amplification, les plages de lyse provoquées par le parasite sont recherchées. Les parasites sont repiqués et leur ADN génomique est extrait pour des analyses PCR. Ces analyses PCR doivent confirmer l'intégration du transgène mais doivent également permettre de différencier les parasites ayant intégré le transgène de manière aléatoire des parasites d'intérêt dont le gène *ncmic3* a été effectivement supprimé par recombinaison homologue.

### c) Analyse PCR

A partir de l'ADN génomique, des PCR ont été réalisées pour :
- étudier la taille du fragment d'ADN amplifié avec un jeu d'amorces de la PCR n°1: HR NCmic3 F (SEQ ID NO: 5) et HR NCmic3 R (SEQ ID NO: 6), présentes sur les séquences homologues. Avec une intégration aléatoire du transgène, deux fragments d'ADN de 2163 pb et de 3824 pb sont amplifiés alors qu'avec recombinaison homologue, seul un fragment de 3824 pb est amplifié. Avec les souches sauvages, seul un fragment de 2163 pb est amplifié.
- vérifier la présence / l'absence du gène *ncmic3* avec le jeu d'amorces de la PCR n°2: ORF NCmic3 F (SEQ ID NO: 7) et ORF NCmic3 R (SEQ ID NO: 8).
- et/ou vérifier la présence / l'absence de la cassette *DHFR* avec le jeu d'amorce de la PCR n°3: ORF DHFR F (SEQ ID NO: 9) et ORF DHFR R (SEQ ID NO: 10).

Les séquences des amorces et la taille des amplicons issus des différentes PCR figurent respectivement dans le Tableau II et le Tableau III ci-dessous.

**Tableau II : Liste des amorces ayant servi aux différentes PCR de validation de la construction de la souche mutante Neo ncmic3 KO.**

| **Nom de l'amorce** | **Séquence 5' → 3'** | **n° de séquence** | **n° de PCR** |
|---|---|---|---|
| HR NCmic3 F | GTCATCGACCGCCGGAACTAGTAGT | SEQ ID NO: 5 | 1 |
| HR NCmic3 R | GCAGAGGTTCTGCGTATCTAACACGG | SEQ ID NO: 6 | 1 |
| ORF NCmic3 F | TTTCCCTTCTAAACACAGTCG | SEQ ID NO: 7 | 2 |
| ORF NCmic3 R | CCTTCAGTGGTTCTCCATGAGT | SEQ ID NO: 8 | 2 |
| ORF DHFR F | CCTTCTCAGACAACGGGGTA | SEQ ID NO: 9 | 3 |
| ORF DHFR R | AGATCTTCACGCCCTTCTCA | SEQ ID NO: 10 | 3 |
| Integ NCmic3 F | GAAAGTGTCAGTGGTAGAGACTGC | SEQ ID NO: 11 | 4 et 6 |
| ORF NCmic3 R2 | CCTTCACTCGAGATCGCGCAAATGAGC | SEQ ID NO: 12 | 4 |
| ORF DHFR R2 | GGACCTCTGTACGAGACATGCCG | SEQ ID NO: 13 | 6 |
| Integ NCmic3 R | TGTTTACAGGTGATCCAGAAAAGG | SEQ ID NO: 14 | 5 et 7 |
| ORF NCmic3 F2 | GAATTTTGGGACAGGGGAAT | SEQ ID NO: 15 | 5 |
| ORF DHFR F2 | GTCTCTCGTTTTCCTCTCTTTTCGG | SEQ ID NO: 16 | 7 |

**Tableau III : Taille des amplicons (en paires de base) des différentes PCR de validation de la construction de la souche mutante Neo ncmic3 KO.**

| **n° de PCR** | **Neo *ncmic3* KO** | ***Neospora caninum* (NC1)** |
|---|---|---|
| 1 | 3824 | 2163 |
| 2 | - | 850 |
| 3 | 504 | - |
| 4 | - | 3127 |
| 5 | - | 3374 |
| 6 | 2890 | - |
| 7 | 3258 | - |

Les profils électrophorétiques des produits PCR sont présentés sur la Figure 3-A. Parmi les clones étudiés, certains clones présentaient une bande spécifique de *DHFR* (PCR 3) mais pas de bande spécifique de *ncmic3* (PCR 2). La PCR n°1 réalisée sur ces clones a mis en évidence une bande de 3824 pb spécifique d'un clone Neo *ncmic3* KO.

De nouvelles analyses PCR ont été effectuées sur ces clones d'intérêt avec de nouveaux jeux d'amorces. Ces PCR, dites d'intégration, permettent de valider le KO génétique en utilisant une amorce présente sur le génome en amont ou en aval des séquences flanquant le gène *ncmic3* et une deuxième amorce présente dans la cassette de sélection (gène *dhfr*) ou dans le gène d'intérêt (*ncmic3*) (Figure 3-B).

Dans la Figure 3-B, les PCR n°4 et n°5 permettent de montrer la présence de *ncmic3* au locus de *ncmic3.* La PCR n°4 est réalisée avec le jeu d'amorce Integ NCmic3 F (SEQ ID NO: 11) et ORF NCmic3 R2 (SEQ ID NO: 12). La PCR n°5 est réalisée avec le jeu d'amorce Integ NCmic3 R (SEQ ID NO: 14) et ORF NCmic3 F2 (SEQ ID NO: 15). On observe la présence de bandes pour la souche sauvage NC1 de *Neospora caninum* et l'absence de ces bandes pour la souche mutante Neo *ncmic3* KO. Dans la Figure 3-B, les PCR n°6 et n°7 permettent de montrer la présence de *DHFR* au locus de *ncmic3.* La PCR n°6 est réalisée avec le jeu d'amorce Integ NCmic3 (SEQ ID NO: 11) et ORF DHFR R2 (SEQ ID NO: 13). La PCR n°7 est réalisée avec le jeu d'amorce Integ NCmic3 R (SEQ ID NO: 14) et ORF DHFR F2 (SEQ ID NO: 16). On constate l'absence de bandes pour la souche sauvage NC1 de *Neospora caninum* et présence de bandes pour la souche Neo *ncmic3* KO. Il faut noter la présence d'une bande aspécifique pour la PCR n°6 à environ 1000 pb.

L'ensemble des résultats de PCR démontre que la recombinaison homologue a bien eu lieu et que la souche mutante Neo *ncmic3* KO est bien délétée du gène *ncmic3.*

### d) Analyse en immunofluoresence

L'analyse en immunofluorescence a été réalisée. 24h avant l'analyse en immunofluorescence, 5x10⁵ parasites sont déposés dans un puits de p24 contentant un coverslip recouvert d'un tapis cellulaire de cellules HFF.

Les cellules infectées par les parasites sont lavées 2 fois au PBS 1X puis fixées au paraformaldéhyde (3,7% dans du PBS 1X) pendant 30 min. Après 3 lavages au PBS 1X, les cellules sont perméabilisées avec une solution de Triton (0.1% dans du PBS 1X) pendant 5 minutes. Après 3 lavages au PBS 1X, une étape de saturation est effectuée avec une solution de PBS 1X/SVF 10% pendant 30min. Les cellules sont ensuite incubées avec l'anticorps primaire dilué dans une solution de PBS/SVF 2% pendant 1 heure, lavées 3 fois puis incubées avec l'anticorps secondaire dilué dans une solution de PBS/SVF 2% pendant 1 heure. Après 2 lavages au PBS1X, les coverslips sont montés sur lame avec de l'immumount et observés au microscope à fluorescence.

L'anticorps primaire utilisé est un anticorps qui permet de détecter l'expression de la protéine NcMIC3 dans le parasite (anticorps primaire : anticorps de lapin anti-mic3 et anticorps secondaire commercial : Alexa fluor® 594 chèvre anti-lapin, Life technologies réf. A-11012).

Pour la souche sauvage NC1 de *Neospora caninum*, on observe une fluorescence rouge au pole apical du parasite révélant la présence de la protéine NcMIC3 (Figure 4A), alors que pour la souche mutante Neo *ncmic3* KO, on n'observe pas de fluorescence au pole apical du parasite démontrant l'absence de la protéine NcMIC3 (Figure 4B).

### Exemple 2 : Construction de la souche mutante Neo ncmic1 KO

### a) Construction du plasmide pNc miclKO-CAT-GFP

Le plasmide pNcMic1KO-CAT-GFP (Figure 2-B) contient une cassette de sélection *CAT-GFP* codant pour une protéine de fusion permettant à la fois la résistance au chloramphénicol (CAT) et une fluorescence verte (GFP : Green Fluorescent Protein). Celle-ci est placée sous le contrôle du promoteur de l'a-tubuline de *Toxoplasma gondii* pour permettre l'expression du gène dans le parasite. De part et d'autre de la cassette, les régions homologues des séquences flanquant le gène *ncmic1* ont été clonées.

La région 3'UTR du gène *ncmic1* a été amplifiée par PCR à partir de l'ADN génomique de la souche NC1 de *Neospora caninum.* Pour l'amplification, les amorces 3 HR NCmic1 F KpnI et 3 HR NCmic1 R HindIII (SEQ ID NO: 17 et SEQ ID NO: 18) permettent l'amplification de la région 3'UTR du gène *ncmic1* et la création de deux sites de restriction qui ont été utilisés pour cloner le fragment 3HR en amont de la cassette de sélection CAT-GFP dans le plasmide pT230 CAT-GFP (KpnI en 5' et HindIII en 3' du fragment de PCR).
La région 5'UTR du gène *ncmic1* a été amplifiée par PCR à partir de l'ADN génomique de la souche NC1 de *Neospora caninum.* Pour l'amplification, les amorces 5 HR NCmic1 F BamHI et 5 HR NCmic1 R NotI (SEQ ID NO: 19 et SEQ ID NO: 20) permettent l'amplification de la région 5'UTR du *gène ncmic1* et la création de deux sites de restriction qui ont été utilisés pour cloner le fragment 5HR en aval de la cassette de sélection CAT-GFP dans le plasmide pT230 3HRNcMic1CAT-GFP (BamHI en 5' et NotI en 3' du fragment de PCR). Les séquences des amorces figurent dans le Tableau IV ci-dessous.

**Tableau IV : Liste des amorces servant à l'intégration des séquences 5'UTR et 3'UTR du gène ncmic 1. Les séquences des sites de restrictions sont soulignées.**

| **Nom de l'amorce** | **Séquence 5' → 3'** | **n° de séquence** |
|---|---|---|
| 3 HR NCmic1 F KpnI | CGCGGTACCAGGCAGAAGTAAAGA AGGTTCCTC | SEQ ID NO: 17 |
| 3 HR NCmic 1 R HindIII | CGCAAGCTTTGATCACGCAAGAAA AGAAGC | SEQ ID NO: 18 |
| 5 HR NCmic1 F BamHI | CGCGGATCCCATTTGTAGATACGGT TGCACAC | SEQ ID NO: 19 |
| 5 HR NCmic1 R NotI | CGCGCGGCCGCACATTCAGACGGC AGAACTCTG | SEQ ID NO: 20 |

### b) Conditions d'électroporation et de sélection

50 µg du plasmide pNcMic1KO-CAT-GFP purifié puis linéarisé par *KpnI* doivent être ajoutés à 5x10⁷ tachyzoïtes NC1 mis en suspension dans le milieu d'électroporation CYTOMIX contenant de l'ATP (3 mM) et du Glutathion (3 mM) (Van den Hoff et al., Nucleic Acid Research, Jun 11; 20(11):2902), et l'électroporation doit être réalisée en cuvette d'écart 4 mm, dans un volume de 800 µL sur un appareil BioRad (Paramètres : 2000 V, 50 ohms, 25 µF, avec deux chocs électriques).

Après électroporation, les tachyzoïtes seront déposés sur une monocouche de cellules HFF en culture. Pour la sélection des mutants, le milieu de culture sera remplacé et supplémenté par l'agent de sélection (chloramphénicol 50 µM), 24h après l'électroporation. Trois passages en culture doivent être effectués dans ce milieu.

Après 15 jours de sélection, les parasites résistants seront clonés par dilution limite dans les puits de plaques de 96 puits de cellules HFF. Après amplification, les plages de lyse provoquées par le parasite seront recherchées. Les parasites seront repiqués et leur ADN génomique sera extrait pour des analyses PCR.

### c) Analyse PCR

Les séquences des amorces et la taille attendue des amplicons issus des différentes PCR figurent respectivement dans le Tableau V et le Tableau VI ci-dessous.

**Tableau V : Liste des amorces servant aux différentes PCR de validation de la construction des souches mutantes Neo ncmic1 KO.**

| **Nom de l'amorce** | **Séquence 5' → 3'** | **n° de séquence** | **n° de PCR** |
|---|---|---|---|
| Integ NCmic1 F | CCGAGCAAGTTAGCAAGTCC | SEQ ID NO: 21 | 1 et 3 |
| ORF CATGFP R | CCGTTTGGTGGATGTCTTCT | SEQ ID NO: 22 | 1 |
| ORF CATGFP F | GCATCGACTTCAAGGAGGAC | SEQ ID NO: 23 | 2 |
| Integ NCmic1 R | CTTGTCCGTCACATCGTTTG | SEQ ID NO: 24 | 2 et 4 |
| ORF NCmic1 R | TTCTCCAGGCACTCACCTCT | SEQ ID NO: 25 | 3 |
| ORF NCmic1 F | AGCTTCCAACAACGAGAGGA | SEQ ID NO: 26 | 4 |
| ORF NCmic1 F2 | CCCAGGATATCGTTTGTTGC | SEQ ID NO: 27 | 5 |
| ORF NCmic1 R2 | CTTCTGATGCACGGAACTGA | SEQ ID NO: 28 | 5 |
| ORF CATGFP F2 | CCTGAAGTTCATCTGCACCA | SEQ ID NO: 29 | 6 |
| ORFCATGFP R2 | GTAGTGGTTGTCGGGCAGCA | SEQ ID NO: 30 | 6 |

**Tableau VI : Taille des amplicons (en paires de base) des différentes PCR de validation de la construction de la souche mutante Neo ncmic1 KO.**

| **n° de PCR** | **Neo *ncmic1*KO** | ***Neospora caninum* (NC1)** |
|---|---|---|
| 1 | 3359 | - |
| 2 | 3421 | - |
| 3 | - | 3746 |
| 4 | - | 3046 |
| 5 | - | 449 |
| 6 | 472 | - |

### Exemple 3 : Construction de la souche mutante Neo ncmic1-3 KO

### a) Construction du plasmide pNc mic1KO CAT-GFP

La construction du plasmide pNcMic1KO-CAT-GFP est décrite dans l'exemple 2 (2a).

### b) Conditions d'électroporation et de sélection

50 µg du plasmide pNcMic1KO-CAT-GFP purifié puis linéarisé par *KpnI* ont été ajoutés à 5x10⁷ tachyzoïtes Neo *ncmic3* KO mis en suspension dans le milieu d'électroporation CYTOMIX contenant de l'ATP (3 mM) et du Glutathion (3 mM) (Van den Hoff et al., Nucleic Acid Research, Jun 11; 20(11):2902), et l'électroporation a été réalisée en cuvette d'écart 4 mm, dans un volume de 800 µL sur un appareil BioRad (Paramètres : 2000 V, 50 ohms, 25 µF, avec deux chocs éléctriques).

Après électroporation, les tachyzoïtes ont été déposés sur une monocouche de cellules HFF en culture. Pour la sélection des mutants, le milieu de culture est remplacé et supplémenté par l'agent de sélection (chloramphénicol 50 µM), 24h après l'électroporation. Trois passages en culture sont effectués dans ce milieu.

Après 15 jours de sélection, les parasites résistants sont clonés par dilution limite dans les puits de plaques de 96 puits de cellules HFF. Après amplification, les plages de lyse provoquées par le parasite sont recherchées. Les parasites sont repiqués et leur ADN génomique est extrait pour des analyses PCR.

### c) Analyse PCR

Les séquences des amorces et la taille des amplicons issus des différentes PCR figurent respectivement dans le Tableau VII et le Tableau VIII ci-dessous.

**Tableau VII : Liste des amorces ayant servi aux différentes PCR de validation de la construction des souches mutantes Neo ncmic3 KO et Neo ncmic1-3 KO.**

| **Nom de l'amorce** | **Séquence 5' → 3'** | **n° de séquence** | **n° de PCR** |
|---|---|---|---|
| Integ NCmic1 F | CCGAGCAAGTTAGCAAGTCC | SEQ ID NO: 21 | 1 et 3 |
| ORF CATGFP R | CCGTTTGGTGGATGTCTTCT | SEQ ID NO: 22 | 1 |
| ORF CATGFP F | GCATCGACTTCAAGGAGGAC | SEQ ID NO: 23 | 2 |
| Integ NCmic1 R | CTTGTCCGTCACATCGTTTG | SEQ ID NO: 24 | 2 et 4 |
| ORF NCmic1 R | TTCTCCAGGCACTCACCTCT | SEQ ID NO: 25 | 3 |
| ORF NCmic1 F | AGCTTCCAACAACGAGAGGA | SEQ ID NO: 26 | 4 |
| Integ NCmic3 F | GAAAGTGTCAGTGGTAGAGACTGC | SEQ ID NO: 11 | 5 et 7 |
| ORF NCmic3 R2 | CCTTCACTCGAGATCGCGCAAATGAGC | SEQ ID NO: 12 | 5 |
| ORF DHFR R2 | GGACCTCTGTACGAGACATGCCG | SEQ ID NO: 13 | 7 |
| Integ NCmic3 R | TGTTTACAGGTGATCCAGAAAAGG | SEQ ID NO: 14 | 6 et 8 |
| ORF NCmic3 F2 | GAATTTTGGGACAGGGGAAT | SEQ ID NO: 15 | 6 |
| ORF DHFR F2 | GTCTCTCGTTTTCCTCTCTTTTCGG | SEQ ID NO: 16 | 8 |
| ORF NCmic1 F2 | CCCAGGATATCGTTTGTTGC | SEQ ID NO: 27 | 9 |
| ORF NCmic1 R2 | CTTCTGATGCACGGAACTGA | SEQ ID NO: 28 | 9 |
| ORF CATGFP F2 | CCTGAAGTTCATCTGCACCA | SEQ ID NO: 29 | 10 |
| ORFCATGFP R2 | GTAGTGGTTGTCGGGCAGCA | SEQ ID NO: 30 | 10 |
| ORF NCmic3 F | TTTCCCTTCTAAACACAGTCG | SEQ ID NO: 7 | 11 |
| ORF NCmic3 R | CCTTCAGTGGTTCTCCATGAGT | SEQ ID NO: 8 | 11 |
| ORF DHFR F | CCTTCTCAGACAACGGGGTA | SEQ ID NO: 9 | 12 |
| ORF DHFR R | AGATCTTCACGCCCTTCTCA | SEQ ID NO: 10 | 12 |

**Tableau VIII : Taille des amplicons (en paires de base) des différentes PCR de validation de la construction des souches mutante Neo ncmic3 KO et Neo ncmic1-3 KO.**

| **n° de PCR** | **Neo *ncmic1-3* KO** | ***Neospora caninum* (NC1)** | **Neo *ncmic3* KO** |
|---|---|---|---|
| 1 | 3359 | - | - |
| 2 | 3421 | - | - |
| 3 | - | 3746 | 3746 |
| 4 | - | 3046 | 3046 |
| 5 | - | 3127 | - |
| 6 | - | 3374 | - |
| 7 | 2890 | - | 2890 |
| 8 | 3258 | - | 3258 |
| 9 | - | 449 | 449 |
| 10 | 472 | - | - |
| 11 | - | 850 | - |
| 12 | 504 | - | 504 |

Dans la Figure 5, la PCR n°1 est réalisée avec le jeu d'amorces Integ NCmic1 F(SEQ ID NO: 21) et ORF CATGFP R (SEQ ID NO: 22). Le PCR 2 est réalisé avec le jeu d'amorces ORF CATGFP F (SEQ ID NO: 23) et Integ NCmic1 R (SEQ ID NO: 24). La PCR n°3 est réalisée avec le jeu d'amorces Integ NCmic1 F (SEQ ID NO: 21) et ORF NCmic1 R (SEQ ID NO: 25). La PCR n°4 est réalisée avec le jeu d'amorces Integ NCmic1 R (SEQ ID NO: 24) et ORF NCmic1 F (SEQ ID NO: 26). La PCR n°5 est réalisée avec le jeu d'amorces Integ NCmic3 F (SEQ ID NO: 11) et ORF NCmic3 R2 (SEQ ID NO: 12). La PCR n°6 est réalisée avec le jeu d'amorces Integ NCmic3 R (SEQ ID NO: 14) et ORF NCmic3 F2 (SEQ ID NO: 15). La PCR n°7 est réalisée avec le jeu d'amorces Integ NCmic3 F (SEQ ID NO: 11) et ORF DHFR R2 (SEQ ID NO: 13). La PCR n°8 est réalisée avec le jeu d'amorces Integ NCmic3 R (SEQ ID NO: 14) et ORF DHFR F2 (SEQ ID NO: 16). La PCR n°9 est réalisée avec le jeu d'amorces ORF NCmic1 F2 (SEQ ID NO: 27) et ORF NCmic1 R2 (SEQ ID NO: 28). La PCR n°10 est réalisée avec le jeu d'amorces ORF CATGFP F2 (SEQ ID NO: 29) et ORF CATGFP R2 (SEQ ID NO: 30). La PCR n°l 1 est réalisée avec le jeu d'amorces ORF NCmic3 F (SEQ ID NO: 7) et ORF NCmic3 R (SEQ ID NO: 8). La PCR n°12 est réalisée avec le jeu d'amorces ORF DHFR F (SEQ ID NO: 9) et ORF DHFR R (SEQ ID NO: 10).

Les analyses électrophorétiques des produits de PCR montrent que la souche Neo *ncmic1-3* KO ne possède plus les gènes *ncmic1* et *ncmic3* (puits 3, 4, 5, 6, 9 et 11, Figure 5) et possède bien les gènes *dhfr* et *cat-gfp* (puits 1, 2, 7, 8, 10 et 12, Figure 5) validant ainsi l'obtention de la souche Neo *ncmic1-3* KO. L'ensemble des résultats de PCR démontre que la recombinaison homologue a bien eu lieu et que la souche Neo *ncmic1-3* KO est bien délétée des gènes *ncmic1* et *ncmic3.*

### d) Analyse en immunofluoresence

L'analyse en immunofluorescence a uniquement été réalisée par observation directe de la fluorescence du parasite (Figure 6).

Les parasites des deux souches mutantes sont visualisés en lumière directe (images A et C). Un même champ microscopique est visualisé en fluorescence. La fluorescence verte, due à l'expression de la protéine recombinante chimérique CAT-GFP n'est détectée que dans la souche mutante Neo *ncmic1-3* KO (image D) suite à l'insertion de la cassette CAT-GFP. A l'inverse, la souche Neo *ncmic3* KO, qui ne possède pas de cassette CAT-GFP, n'exprime pas la protéine CAT-GFP et par conséquent ne présente pas de fluorescence (image B).

### Exemple 4 : Effets de l'inactivation de la protéine NcMIC3 ou des protéines NcMIC1 et NcMIC3 sur les propriétés infectieuses de Neospora caninum

Les souches mutantes Neo *ncmic3* KO et Neo *ncmic1-3* KO décrits dans les exemples 1 et 3 ont été entretenues par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct ;133(4):421-32), le nombre de passages sur cellules HFF est volontairement limité à 20.

Entre 60 et 80% des souris femelles Balb/C meurent généralement entre 8 et 11 jours après une infection par voie intrapéritonéale par 10⁷ tachyzoïtes de la souche NC1 *de Neospora caninum.*

L'étude de la virulence des mutants Neo *ncmic3* KO et Neo *ncmic1-3* KO a été étudiée sur un lot minimal de 10 souris femelles Balb/C par injection intrapéritonéale de 10⁷ tachyzoïtes/souris. Les contrôles ont été réalisés dans les mêmes conditions sur des lots de 10 souris femelles Balb/C en utilisant la souche NC1 de *Neospora caninum.*

La Figure 7 montre que 70% des souris infectées avec 10⁷ tachyzoïtes de la souche NC1 de *Neospora caninum* sont mortes 11 jours après l'infection (ronds noirs). Les souris infectées par la souche mutante Neo *ncmic3* KO (carrés noirs) présentent un retard dans la mortalité (mort des souris entre 9 jours et 17 jours après l'infection) et une atténuation significative de la virulence du parasite (30% de mortalité contre 70% avec la souche sauvage 29 jours après l'infection). De plus, dans le cas des souris infectées par la souche mutante Neo *ncmic1-3* KO (triangles noirs), on observe 100% de survie 29 jours après l'infection.

Les souris ont également été infectées avec des quantités croissantes de la souche NC1 de *Neospora caninum* et les souches mutantes Neo *ncmic3* KO et Neo *ncmic1-3* KO. La dose nécessaire pour provoquer 50% de mortalité (DL50) est de 6x10⁶ tachyzoïtes pour la souche sauvage NC1 de *Neospora caninum* et de 22x10⁶ tachyzoïtes pour la souche Neo *ncmic3* KO. Pour la souche Neo *ncmic1-3* KO, la DL50 est très largement supérieure à 10⁸ tachyzoïtes soit 17 fois la DL50 de la souche sauvage NC1 de *Neospora caninum.* En effet, aucune mortalité n'est observée à cette dose avec la souche mutante Neo *ncmic1-3* KO.

### Exemple 5 : Efficacité de la souche Neo ncmic1-3 KO dans la prévention de la néosporose dans un modèle murin de néosporose létale

La souche mutante Neo *ncmic1-3* KO décrite à l'Exemple 3 a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct;133(4):421-32), le nombre de passage sur cellules HFF est volontairement limité à 20.

Des souris Balb/C femelles ont été séparées en 6 lots distincts : (i) un lot vacciné par voie intrapéritonéale avec 5x10⁶ tachyzoïtes de la souche mutante Neo *ncmic1-3* KO, (ii) un lot vacciné par voie intrapéritonéale avec 10⁷ tachyzoïtes de la souche mutante Neo *ncmic1-3* KO, (iii) un lot vacciné par voie intrapéritonéale avec 10⁷ tachyzoïtes de la souche mutante Neo *ncmic1-3* KO et boosté 1 mois après la première injection par 10⁷ tachyzoïtes de la souche mutante Neo *ncmic1-3* KO, (iv) un lot vacciné par voie intrapéritonéale avec 5x10⁷ tachyzoïtes de la souche mutante Neo *ncmic1-3* KO, (v) un lot vacciné par voie intrapéritonéale avec 10⁸ tachyzoïtes de la souche mutante Neo *ncmic1-3* KO et (vi) un lot témoin non vacciné.

Quatre mois après la vaccination, toutes les souris ont été challengées par voie intrapéritonéale avec 2x10⁷ tachyzoïtes de la souche sauvage NC1 de *Neospora caninum.* La souche sauvage NC1 de ***Neospora** caninum* a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov;7(6)893-898 and Bartley et al., Parasitology, 2006, Oct ;133(4):421-32), le nombre de passages sur cellules HFF est volontairement limité à 20. La dose utilisée pour le challenge est suffisante pour entraîner 100% de mortalité chez les souris challengées. La survie des souris est ensuite suivie pendant une durée d'un mois.

La Figure 8 montre que les souris vaccinées des lots (i) à (iv) sont totalement protégées d'une réinfection par une souche virulente sauvage NC1 de ***Neospora** caninum* provoquant 100% de mortalité chez les souris du lot témoin (vi) qui meurent toutes au 6^{ème} jour après le challenge. Les souris vaccinées avec la plus forte dose de la souche Neo *ncmic1-3* KO (lot (v)) présentent une mortalité intermédiaire (50%). Contrairement aux souris du lot témoin (vi), la mortalité des souris de ce lot intervient de manière plus précoce (4,5 jours en moyenne). Cette observation pourrait s'expliquer par une réponse inflammatoire forte générée par ce groupe de souris au moment du challenge.

### Exemple 6 : Efficacité de la souche mutante Neo ncmic1-3 KO dans la prévention de la néosporose dans un modèle murin de néosporose congénitale - Expérience 1

La souche mutante Neo *ncmic1-3* KO décrite à l'Exemple 3 a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct ;133(4):421-32), le nombre de passages sur cellules HFF est volontairement limité à 20.

Des souris OF1 femelles ont été séparées en 2 lots distincts : (i) un lot vacciné par voie intrapéritonéale avec 10⁷ tachyzoïtes de la souche mutante Neo *ncmic1-3* KO, (ii) un lot témoin non vacciné.

Deux mois après vaccination, les souris ont été mises au mâle (J0) à raison de trois souris femelles pour un mâle. Les souris gestantes sont diagnostiquées par pesée et sont soumises au dixième jour de gestation à un challenge infectieux par voie intrapéritonéale avec 2x10⁶ tachyzoïtes de la souche sauvage NC1 de ***Neospora** caninum.* La souche sauvage NC1 de ***Neospora** caninum* a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov;7(6)893-898 and Bartley et al., Parasitology, 2006, Oct ;133(4):421-32), le nombre de passages sur cellules HFF est volontairement limité à 20.

Un jour avant la mise bas, les souris femelles ont été sacrifiées. Les placentas et les foetus ont été isolés et l'ADN est extrait. Une PCR nichée est réalisée à partir de la région du gène *NC5* de *N. caninum* (Yamage et al., J.Parasitol,1996 Apr,82(2): 272-9, Baszler et al.,J Clin Microbiol, 1999 Dec,37(12): 4059-64). Pour la PCR primaire, le couple d'amorces NC5 FA (SEQ ID NO : 31) et NC5 RA (SEQ ID NO : 32) est utilisé et le couple d'amorces NC5 FB (SEQ ID NO : 33) et NC5 RB (SEQ ID NO : 34) est utilisé pour la PCR secondaire. Les séquences des amorces figurent dans le Tableau IX ci-dessous.

**Tableau IX : Liste des amorces utilisées pour les PCR de recherche de la présence du parasite N. caninum dans les tissus.**

| **Nom de l'amorce** | **Séquence 5' → 3'** | **n° de séquence** | **n° de PCR** |
|---|---|---|---|
| NC5 FA | CCCAGTGCGTCCAATCCTGTAAC | SEQ ID NO: 31 | Primaire |
| NC5 RA | CTCGCCAGTCAACCTACGTCTTCT | SEQ ID NO: 32 | Primaire |
| NC5 FB | TAATCTCCCCCGTCATCAGT | SEQ ID NO: 33 | Secondaire |
| NC5 RB | GGGTGAACCGAGGGAGTTG | SEQ ID NO: 34 | Secondaire |

Pour chaque placenta et foetus, trois PCR indépendantes sont réalisées. Les placentas et les foetus sont considérés positifs lorsque ***Neospora** caninum* est détecté pour au moins une PCR. Les résultats sont présentés dans le Tableau X ci-dessous.

**Tableau X : Recherche de Neospora caninum dans les tissus placentaires et foetaux de souris vaccinées avec la souche Neo ncmic1-3 KO et challengées avec la souche sauvage NC1 (lot (i)) en comparaison avec des souris témoin non vaccinées et challengées avec la souche sauvage NC1 (lot (ii)).**

| | | | |
|---|---|---|---|
| **Lot (i)** Souris vaccinées avec 10⁷ tachyzoïtes de la souche Neo *ncmic1-3* KO et challengées avec 2.10⁶ tachyzoïtes de la souche sauvage NC1 | **Recherche de *Neospora caninum* dans les placentas** | | |
| | Nombre de placentas étudiés | Nombre de placentas positifs | % de placentas positifs |
| | 106 | 47 | 44,3% |
| | **Recherche de *Neospora caninum* dans les foetus** | | |
| | Nombre de foetus étudiés | Nombre de foetus positifs | % de foetus positifs |
| | 109 | 23 | 21,1% |
| **Lot (ii)** Souris non vaccinées et challengées avec 2.10⁶ tachyzoïtes de la souche sauvage NC1 | **Recherche de *Neospora caninum* dans les placentas** | | |
| | Nombre de placentas étudiés | Nombre de placentas positifs | % de placentas positifs |
| | 37 | 37 | 100% |
| | **Recherche de *Neospora caninum* dans les foetus** | | |
| | Nombre de foetus étudiés | Nombre de foetus positifs | % de foetus positifs |
| | 36 | 27 | 75% |

Ces résultats démontrent que la vaccination avec la souche mutante atténuée Neo *ncmic1-3* KO réduit considérablement la transmission materno-foetale du parasite, validant ainsi l'efficacité de la souche Neo *ncmic1-3* KO à prévenir des effets délétères de la néosporose dans un modèle murin de néosporose congénitale endogène.

### Exemple 7 : Efficacité de la souche mutante Neo ncmic1-3 KO dans la prévention de la néosporose dans un modèle ovin de néosporose congénitale

### a) Déroulement de l'expérimentation

La souche mutante Neo *ncmic1-3* KO décrits à l'Exemple 3 a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct ;133(4):421-32), le nombre de passages sur cellules HFF est volontairement limité à 20.

Des brebis Romanov séronégatives pour ***Neospora** caninum* et pour *Toxoplasma gondii* ont été séparées en 4 lots distincts : un lot composé de 14 brebis témoin non vaccinées par la souche mutante Neo *ncmic1-3* KO et challengées par voie sous-cutanée avec 10⁷ tachyzoïtes de la souche sauvage NC1 de ***Neospora** caninum* (lot i), un lot composé de 15 brebis vaccinées par voie sous-cutanée avec 10⁷ tachyzoïtes de la souche mutante Neo *ncmic1-3* KO puis boostées par voie sous-cutanée 1 mois après la première injection par 10⁷ tachyzoïtes de la souche mutante Neo *ncmic1-3* KO et challengées par voie sous-cutanée avec 10⁷ tachyzoïtes de la souche sauvage NC1 de *Neospora caninum* (lot ii), un lot composé de 14 brebis vaccinées par voie sous-cutanée avec 10⁸ tachyzoïtes de la souche mutante Neo *ncmic1-3* KO et challengées par voie sous-cutanée avec 10⁷ tachyzoïtes de la souche sauvage NC1 de ***Neospora** caninum* (lot iii) et un lot composé de 5 brebis témoin non vaccinées par la souche mutante Neo *ncmic1-3* KO et non challengées par la souche sauvage NC1 de ***Neospora** caninum* (lot iv).

Deux mois après la première vaccination, les brebis ont été inséminées artificiellement. Un retour au bélier a été entrepris 3 semaines après l'insémination artificielle. Des échographies ont ensuite été réalisées et ont permis de diagnostiquer que 14 brebis sur 14 étaient gestantes dans le lot (i), 13 brebis sur 15 dans le lot (ii),13 brebis sur 14 dans le lot (iii) et 4 brebis sur 5 dans le lot (iv).

Les brebis gestantes des lots (i), (ii) et (iii) ont été soumises à mi-gestation à un challenge infectieux avec 10⁷ tachyzoïtes de la souche sauvage NC1 de ***Neospora** caninum.* La souche sauvage NC1 de ***Neospora** caninum* a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct ;133(4):421-32), le nombre de passages sur cellules HFF est volontairement limité à 20.

### b) Relevé de température post-immunisation et post-challenge

De 5 jours avant la vaccination à 14 jours post-vaccination, les températures rectales des brebis ont été enregistrées quotidiennement. Les moyennes des températures post-immunisation des lots (i), (ii) (iii) et (iv) sont présentées dans la Figure 12-A. Les températures des lots témoins (iii) et (iv) restent physiologiques. En revanche, un pic thermique est observé pour les deux lots vaccinés (> 39,5°C). Un retour aux températures physiologiques est observé 5 jours après l'immunisation.

Le jour avant l'infection et pendant les jours suivants, la température rectale a été relevée quotidiennement. Les moyennes des températures post-infection des lots (i), (ii) (iii) et (iv) sont présentés dans la Figure 12-B. Les températures du lot témoin (iv) restent physiologiques. En revanche, un pic thermique est observé pour les lots vaccinés (i), (ii) et (iii). Pour le lot témoin uniquement infecté (lot (i)), le pic fébrile a duré 3 jours avec un maximum à 40°C à J3. Pour les lots vaccinés (ii) et (iii), le pic fébrile intervient dès J2, ne dure que deux jours et est moins intense (39,5°C).

### c) Analyse de la réponse immunitaire humorale

La réponse immunitaire a été étudiée post-immunisation et post-challenge en évaluant par ELISA la cinétique d'apparition des IgG spécifiques anti-*N*. *caninum* dans le sérum des brebis des lots (i), (ii) (iii) et (iv). Les sérums sont prélevés avant l'immunisation (J0) puis à J22, J57 et J107 post-vaccination et enfin après challenge (J0 Chal, J29 Chal, J62 Chal). Le sang est prélevé au niveau de la veine jugulaire et le prélèvement est laissé une nuit à 4°C pour permettre la formation du caillot. Le sérum est récupéré en centrifugeant les prélèvements à 5000 g pendant 10 min. Le surnageant est récupéré et conservé à -20°C.

Pour analyser la réponse immunitaire humorale induite après la vaccination, un extrait de *N. caninum* est préparé. Pour la préparation de cet extrait parasitaire total, les tachyzoïtes de la souche NC-1 sont lavés, soniqués deux fois à 60 watts/s durant 10 min dans la glace et centrifugés à 2 000 g pendant 30 minutes à +4°C. Le surnagenant est récupéré et la concentration est détérminée par un dosage BCA qui utilise comme standard la Sérum Albumine Bovine (SAB). Les aliquots sont conservés à -80°C jusqu'à utilisation.

L'extrait parasitaire total de la souche NC1 est dilué dans un tampon carbonate pH9,6 pour obtenir une concentration finale de 10 µg/mL. Les plaques sont ensuite lavées trois fois avec le tampon de lavage (PBS 1X - Tween 20 0,05%) puis saturées pendant 1h30 à 37°C avec une solution de PBS 1X - Tween 20 0,05% complémentée avec 4% de Sérum Albumine Bovine (SAB) (Sigma). Le milieu est ensuite éliminé. Les sera à tester sont dilués au 1/50ème dans une solution de PBS 1X - Tween 20 0,05% et sont déposés en duplicate dans les puits. Après une heure d'incubation à 37°C et une nouvelle série de lavage, l'anticorps secondaire anti-IgG de mouton couplé à la phosphatase alcaline (Jackson ImmunoResearch 713-055_147, donkey anti-Sheep IgG) et dilué au 1/5000ème est déposé à raison de 100 µL par puits. Les échantillons sont alors incubés pendant une heure à 37°C. Après une nouvelle série de trois lavages, la révélation est faite par l'ajout dans chaque puits de 100 µL d'une solution de paranitrophénylphosphate disodique (PnPP) (Sigma) à 1 mg/mL, dans un tampon DEA-HCl. Après 20 min d'incubation à température ambiante et à l'abri de la lumière, l'absorbance à 405 nm est mesurée grâce à un lecteur de plaque (Multiskan MCC340 Wallace). Les moyennes des résultats des tests ELISA à J0, J22, J57 et J107 post-immunisation et à J0, J29, J62 post-challenge pour les sérums des différents lots de brebis dilués au 1/50eme sont représentés dans la Figure 13.

Après immunisation, les brebis des lots témoins (i) et (iv) non vaccinées n'ont pas développé de réponse humorale. En revanche, les brebis des lots (ii) et (iii) ont développé une réponse IgG anti-néosporose dès J22. Cette réponse IgG est boosté lors de la seconde vaccination du lot (ii). Elle diminue ensuite pour les deux lots (ii) et (iii).

Après challenge, les brebis du lot témoin (iv) non challengé n'ont pas développé de réponse humorale. En revanche, les brebis des lots (i) (ii) et (iii) ont développé une réponse IgG anti-néosporose. La réponse humorale des lots vaccinées (ii) et (iii) est plus rapide que pour le lot (i) non vacciné.

### d) Etude des avortements

Après challenge, les brebis ont été suivies quotidiennement jusqu'à la mise-bas et les avortements et la mortinatalité ont été enregistrés.

Les résultats de cette étude sont illustrés dans le Tableau XV ci-dessous.

| **Lots** | **Nombre d'agneaux attendus (%)** | **Nombre d'avortement (%)** | **Nombre de mort-nés (%)** | **Nombre d'agneaux vivants (%)** |
|---|---|---|---|---|
| (i) (Non vacciné / Infecté) | 35 (100%) | 29 (82,9%) | 1(2,8%) | 5 (2,8%) |
| (ii) (vacciné puis boosté avec 10⁷ tachyzoïtes puis infecté) | 38 (100%) | 0(0%) | 11 (29%) | 27(71%) |
| (iii) (vacciné avec 10⁸ tachyzoïtes puis infecté) | 33 (100%) | 3 (9,1%) | 8 (24,2%) | 22 (66,6%) |
| (iv) (non vacciné / non infecté) | 13 (100%) | 0(0%) | 1 (7,7%) | 12 (92,3%) |

Ces résultats démontrent que la vaccination avec la souche mutante atténuée Neo *ncmic1-3* KO réduit considérablement les effets délétères d'une infection d'un ruminant, notamment un ovin, par *Neospora caninum.*

### Exemple 8 : Analyse de la réponse immunitaire humorale suite à la vaccination par la souche Neo ncmic1-3 KO

La souche mutante Neo *ncmic1-3* KO décrits à l'Exemple 3 a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct ;133(4):421-32), le nombre de passages sur cellules HFF est volontairement limité à 20.

Des souris OF1 femelles ont été séparées en 2 lots distincts : (i) un lot vacciné par voie intrapéritonéale avec 5.10⁷ tachyzoïtes de la souche mutante Neo *ncmic1-3* KO, (ii) un lot témoin non vacciné mais challengé.

Un mois après la vaccination, une prise de sang sous-maxillaire est réalisée. Le sang total est conservé 2 heures à 37°C avant d'être centrifugé à 5 000 g pendant 10 minutes afin de conserver le sérum. Le sérum est conservé à -20°C jusqu'à utilisation.

Pour analyser la réponse immunitaire humorale induite après la vaccination, un extrait de *N. caninum* est préparé. Pour la préparation de cet extrait parasitaire total, les tachyzoïtes de la souche NC-1 sont lavés, soniqués deux fois à 60 watts/s durant 10 min dans la glace et centrifugés à 2 000 g pendant 30 minutes à +4°C. Le surnagenant est récupéré et la concentration est déterminée par un dosage BCA qui utilise comme standard la Sérum Albumine Bovine (SAB). Les aliquots sont conservés à -80°C jusqu'à utilisation.

A partir des sérums de souris vaccinés par la souche mutante Neo *ncmic1-3* KO et de souris non vaccinées, des tests ELISA sont réalisés afin de caractériser la réponse immunitaire humorale induite par la souche mutante Neo *ncmic1-3* KO.

### a) Recherche des IgG totales spécifiques de N. caninum

L'extrait parasitaire total de la souche NC1 est dilué dans un tampon carbonate pH9,6 pour obtenir une concentration finale de 10 µg/mL. Des plaques 96 puits à fond plat sont alors sensibilisées une nuit à +4°C par un dépôt dans chaque puits de 100 µL d'extrait total de *N. caninum.* Les plaques sont ensuite lavées trois fois avec le tampon de lavage (PBS 1X - Tween 20 0,05%) puis saturées pendant 1h30 à 37°C avec une solution de PBS 1X - Tween 20 0,05% complémentée avec 4% de Sérum Albumine Bovine (SAB) (Sigma). Le milieu est ensuite éliminé. Les sera à tester sont dilués au 1/50ème dans une solution de PBS 1X - Tween 20 0,05% et sont déposés en duplicate dans les puits. Après une heure d'incubation à 37°C et une nouvelle série de lavage, l'anticorps secondaire anti-IgG de souris couplé à la phosphatase alcaline (Sigma A3562, goat anti-Mouse IgG) et dilué au 1/5000ème est déposé à raison de 100 µL par puits. Les échantillons sont alors incubés pendant une heure à 37°C. Après une nouvelle série de trois lavages, la révélation est faite par l'ajout dans chaque puits de 100 µL d'une solution de paranitrophénylphosphate disodique (PnPP) (Sigma) à 1 mg/mL, dans un tampon DEA-HCl. Après 20 min d'incubation à température ambiante et à l'abri de la lumière, l'absorbance à 405 nm est mesurée grâce à un lecteur de plaque (Multiskan MCC340 Wallace). Les souris sont considérées comme séroconverties lorsque l'absorbance obtenue est 2,5 fois supérieure à l'absorbance obtenue avec le témoin négatif issu de sérum de souris naïves saines (Figure 10).

Les souris vaccinées du lot (i) présentent toutes une séroconversion contrairement aux souris non vaccinées du lot (ii).

### b) Profil isotypique des IgG anti-N. caninum

L'extrait parasitaire total de la souche NC-1 est dilué dans un tampon carbonate pH9,6 pour obtenir une concentration finale de 10 µg/mL. Des plaques 96 puits à fond plat sont alors sensibilisées une nuit à +4°C par un dépôt dans chaque puits de 100 µL d'extrait total de *N. caninum.* Les plaques sont ensuite lavées trois fois avec le tampon de lavage (PBS 1X - Tween 20 0,05%) puis saturées pendant 1h30 à 37°C avec une solution de PBS 1X - Tween 20 0,05% complémentée avec 4% de Sérum Albumine Bovine (SAB) (Sigma). Le milieu est ensuite éliminé. Les sera à tester sont dilués au 1/100^{ème} dans une solution de PBS 1X - Tween 20 0,05% et sont déposés en duplicate dans les puits. Après une heure d'incubation à 37°C et une nouvelle série de lavage, les anticorps secondaires sont déposés. Les anticorps secondaires anti-IgG1 (BD 557272, rat anti-Mouse IgG1) et anti-IgG2a (BD 553389, rat anti-Mouse IgG2a) couplés à la phosphatase alcaline et dilués au 1/1000^{ème} sont déposés à raison de 100 µL par puits. Les échantillons sont alors incubés pendant une heure à 37°C. Après une nouvelle série de trois lavages, la révélation est faite par l'ajout dans chaque puits de 100 µL d'une solution de paranitrophénylphosphate disodique (PnPP) (Sigma) à 1 mg/mL, dans un tampon DEA-HCl. Après 20 min d'incubation à température ambiante et à l'abri de la lumière, l'absorbance à 405 nm est mesurée grâce à un lecteur de plaque (Multiskan MCC340 Wallace) (Figure 11).

Les IgG anti-*N*. *caninum* des souris vaccinées du lot (i) sont préférentiellement de type IgG2A, profil isotypique favorable à une protection contre ***Neospora** caninum* (Long et al., J. Parasitol., 1998, Apr; 84(2):316-20).

### Exemple 9 : Efficacité de la souche mutante Neo mic1-3 KO dans la prévention de la néosporose dans un modèle murin de néosporose congénitale - Expérience 2

La souche mutante Neo *ncmic1-3* KO décrite à l'Exemple 3 a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct ;133(4):421-32), le nombre de passages sur cellules HFF est volontairement limité à 20.

Des souris OF1 femelles ont été séparées en 2 lots distincts : (i) un lot de 11 souris vaccinées par voie intrapéritonéale avec 5.10⁷ tachyzoïtes de la souche mutante Neo *ncmic1-3* KO et (ii) un lot de 6 souris témoins non vaccinées.

Deux mois après vaccination, les souris ont été mises au mâle (J0) à raison de trois souris femelles pour un mâle. Les souris gestantes des lots (i) et (ii) sont diagnostiquées par pesée et sont soumises au dixième jour de gestation à un challenge infectieux par voie intrapéritonéale avec 2x10⁶ tachyzoïtes de la souche sauvage NC1 de *Neospora caninum.* La souche sauvage NC1 de *Neospora caninum* a été entretenue par passages réguliers sur cellules HFF cultivées en milieu DMEM supplémenté par 10% de sérum de veau foetal (FCS), 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000, Nov ;7(6)893-898 and Bartley et al., Parasitology, 2006, Oct ;133(4):421-32), le nombre de passages sur cellules HFF est volontairement limité à 20.

Un jour avant la mise bas, les souris femelles ont été sacrifiées. Les placentas et les foetus ont été isolés et l'ADN est extrait. Une PCR nichée est réalisée à partir de la région du gène *NC5* de *N. caninum* (Yamage et al., J.Parasitol,1996 Apr,82(2): 272-9, Baszler et al., J Clin Microbiol, 1999 Dec,37(12): 4059-64). Pour la PCR primaire, le couple d'amorces NC5 FA (SEQ ID NO : 31) et NC5 RA (SEQ ID NO : 32) est utilisé et le couple d'amorces NC5 FB (SEQ ID NO : 33) et NC5 RB (SEQ ID NO : 34) est utilisé pour la PCR secondaire. Les séquences des amorces figurent dans le Tableau XI ci-dessous.

**Tableau XI : Liste des amorces utilisées pour les PCR de recherche de la présence du parasite N. caninum dans les tissus.**

| **Nom de l'amorce** | **Séquence 5' → 3'** | **n° de séquence** | **n° de PCR** |
|---|---|---|---|
| NC5 FA | CCCAGTGCGTCCAATCCTGTAAC | SEQ ID NO: 31 | Primaire |
| NC5 RA | CTCGCCAGTCAACCTACGTCTTCT | SEQ ID NO: 32 | Primaire |
| NC5 FB | TAATCTCCCCCGTCATCAGT | SEQ ID NO: 33 | Secondaire |
| NC5 RB | GGGTGAACCGAGGGAGTTG | SEQ ID NO: 34 | Secondaire |

Pour chaque placenta et foetus, trois PCR indépendantes sont réalisées. Les placentas et les foetus sont considérés positifs lorsque *Neospora caninum* est détecté pour au moins une PCR. Les résultats sont présentés dans le Tableau XII ci-dessous.

**Tableau XII : Recherche de Neospora caninum dans les tissus placentaires et foetaux de souris vaccinées avec la souche Neo ncmic1-3 KO et challengées avec la souche sauvage NC1 (lot (i)) en comparaison avec des souris témoin non vaccinées et challengées avec la souche sauvage NC1 (lot (ii)).**

| | | | |
|---|---|---|---|
| **Lot (i)** Souris vaccinées avec 5.10⁷ tachyzoïtes de la souche Neo *ncmic1-3* KO et challengées avec 2.10⁶ tachyzoïtes de la souche sauvage NC1 | **Recherche de *Neospora caninum* dans les placentas** | | |
| | Nombre de placentas étudiés | Nombre de placentas positifs | % de placentas positifs |
| | 131 | 15 | 11,4% |
| | **Recherche de *Neospora caninum* dans les foetus** | | |
| | Nombre de foetus étudiés | Nombre de foetus positifs | % de foetus positifs |
| | 142 | 7 | 4,93% |
| **Lot (ii)** Souris non vaccinées et challengées avec 2.10⁶ tachyzoïtes de la souche sauvage NC1 | **Recherche de *Neospora caninum* dans les placentas** | | |
| | Nombre de placentas étudiés | Nombre de placentas positifs | % de placentas positifs |
| | 88 | 82 | 93,2% |
| | **Recherche de *Neospora caninum* dans les foetus** | | |
| | Nombre de foetus étudiés | Nombre de foetus positifs | % de foetus positifs |
| | 87 | 52 | 59,8% |

Ces résultats démontrent que la vaccination avec la souche mutante atténuée Neo *ncmic1-3* KO réduit considérablement la transmission materno-foetale du parasite, validant ainsi l'efficacité de la souche Neo *mic1-3* KO à prévenir des effets délétères de la néosporose dans un modèle murin de néosporose congénitale endogène.

### Exemple 10 : Efficacité de la souche mutante Neo ncmic1-3 KO dans la prévention de la néosporose dans un modèle murin de néosporose congénitale après infection des mères avant gestation

La souche mutante Neo *ncmic1-3* KO décrit à l'Exemple 3 a été entretenue par passages réguliers sur cellules HFF cultivées en milieu DMEM supplémenté par 10% de sérum de veau foetal (FCS) ; 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000 Nov ; 7(6)893-898 et Bartley et al., Parasitology, 2006, Oct ; 133(4) :421-32), le nombre de passages sur cellules HFF est volontairement limité à 20.

Des souris OF1 femelles ont été séparées en 3 lots distincts :
- Lot (i) : lot témoin composé de 9 souris infectées par voie intra-péritonéale avec 5.10⁶ tachyzoites de la souche NC-1,
- Lot (ii) : lot composé de 9 souris infectées par voie intra-péritonéale avec 5.10⁶ tachyzoites de la souche sauvage NC-1 puis vaccinées 58 jours plus tard avec 5.10⁷ tachyzoïtes de la souche Neo *ncmic1-3* KO par voie intra-péritonéale,
- Lot (iii) : lot composé de 9 souris vaccinées par voie intra-péritonéale avec 5.10⁷ tachyzoïtes de la souche Neo *ncmic1-3* KO puis infectées, 58 jours plus tard, par voie intra-péritonéale avec 5.10⁶ tachyzoites de la souche sauvage NC-1.

Cent sept jours après le début de l'expérimentation, les souris ont été mise au mâle à raison de 3 souris femelles pour un mâle. Les souris gestantes sont diagnostiquées par pesée.

A 18 jours de gestation, soit un jour avant les mises-bas théoriques, les souris femelles sont sacrifiées. Les foetus sont isolés et l'ADN est extrait.

Une PCR nichée est réalisée à partir de la région du gène NC5 de *N. caninum* (Yamage et al., J. Parasitol, 1996 Apr, 82(2): 272-9, Bazler et al., J. Clin. Microbiol, 1999 Dec,37(12): 4059-64). Pour la PCR primaire, le couple d'amorces NC5 FA (SEQ ID NO : 31) et NC5 RA (SEQ ID NO : 32) est utilisé et le couple d'amorces NC5 FB (SEQ ID NO : 33) et NC5 RB (SEQ ID NO : 34) est utilisé pour la PCR secondaire. Les séquences des amorces figurent dans le Tableau XIII ci-dessous.

**Tableau XIII : Liste des amorces utilisées pour les PCR de recherche de la présence du parasite N. caninum dans les tissus.**

| **Nom de l'amorce** | **Séquence 5' → 3'** | **n° de séquence** | **n° de PCR** |
|---|---|---|---|
| NC5 FA | CCCAGTGCGTCCAATCCTGTAAC | SEQ ID NO: 31 | Primaire |
| NC5 RA | CTCGCCAGTCAACCTACGTCTTCT | SEQ ID NO: 32 | Primaire |
| NC5 FB | TAATCTCCCCCGTCATCAGT | SEQ ID NO: 33 | Secondaire |
| NC5 RB | GGGTGAACCGAGGGAGTTG | SEQ ID NO: 34 | Secondaire |

Pour chaque foetus, 3 PCR indépendantes sont réalisées. Le foetus est considéré comme positif lorsque *Neospora caninum* est détécté pour au moins une PCR. Les résultats sont présentés dans le Tableau XIV ci-dessous.

**Tableau XIV : Recherche de Neospora caninum dans les tissus placentaires et foetaux de souriceaux nés de mères infectées avant gestation (Lot (i)), de mères infectées puis vaccinées avant gestation (Lot (ii)) et de mères vaccinées puis infectées avant gestation (Lot (iii).**

| | | | |
|---|---|---|---|
| **Lot (i)** 9 souris infectées avec 5.10⁶ tachyzoïtes de la souche sauvage NC1 avant la mise au mâle | **Recherche de *Neospora caninum* dans les placentas** | | |
| | Nombre de placentas étudiés | Nombre de **placentas** positifs | % de placentas positifs |
| | 96 | 28 | 29,2% |
| | **Recherche de *Neospora caninum* dans les foetus** | | |
| | Nombre de foetus étudiés | Nombre de foetus positifs | % de foetus positifs |
| | 97 | 32 | 33% |
| **Lot (ii)** 9 souris infectées avec 5.10⁶ tachyzoïtes de la souche sauvage NC1 puis vaccinées avec 5.107 tachyzoïtes de la souche Neo *mic1-3* KO avant la mise au mâle | **Recherche de *Neospora caninum* dans les placentas** | | |
| | Nombre de placentas étudiés | Nombre de **placentas** positifs | % de placentas positifs |
| | 110 | 13 | 11,81% |
| | **Recherche de *Neospora caninum* dans les foetus** | | |
| | Nombre de foetus étudiés | Nombre de foetus positifs | % de foetus positifs |
| | 113 | 15 | 13,27% |
| Lot (ii) 9 souris vaccinées avec 5.10⁷ tachyzoïtes de la souche Neo mic1-3 KO puis infectées avec 5.10⁶ tachyzoïtes de la souche sauvage NC1 avant la mise au mâle | **Recherche de *Neospora caninum* dans les placentas** | | |
| | Nombre de placentas étudiés | Nombre de **placentas** positifs | % de plancetas positifs |
| | 88 | 20 | 22,72% |
| | **Recherche de *Neospora caninum* dans les foetus** | | |
| | Nombre de foetus étudiés | Nombre de foetus positifs | % de foetus positifs |
| | 88 | 13 | 14,7% |

Ces résultats démontrent que la vaccination avec la souche mutante atténuée *Neo ncmic1-3 KO* réduit significativement (Test de Chi2, p<0,05) la transmission materno-foetale du parasite lorsque les mères sont infectées avant la gestation validant ainsi l'efficacité prophylactique et thérapeutique de la souche *Neo ncmic1-3 KO* à prévenir des effets délétères de la néosporose dans un modèle murin de néosporose congénitale avec infection de la mère avant la gestation.

### Exemple 11 : Test diagnostic permettant de différencier la souche vaccinale Neo mic1-3 KO de la souche sauvage NC1 après injection à des souris.

La souche mutante Neo *ncmic1-3* KO décrite à l'Exemple 3 a été entretenue par passages réguliers sur cellules HFF cultivées en milieu DMEM supplémenté par 10% de sérum de veau foetal (FCS) ; 2 mM glutamine, 50 U/mL de pénicilline et 50 µg/mL de streptomycine. Les passages sur cellules HFF réduisant la virulence des parasites (Baszler et al., Clin. Diagn. Lab. Immunol., 2000 Nov ; 7(6)893-898 et Bartley et al., Parasitology, 2006, Oct ; 133(4) :421-32), le nombre de passages sur cellules HFF est volontairement limité à 20.

Des souris Balb/c femelles ont été séparées en 2 lots distincts :
- un lot injecté par voie intrapéritonéale avec 5.10⁷ tachyzoïtes de la souche mutante Neo *ncmic1-3* KO,
- et un lot injecté par voie intrapéritonéale avec 10⁷ tachyzoïtes de la souche sauvage NC1 de ***Neospora** caninum.*

Les souris ont été sacrifiées. Le cerveau des souris est ensuite prélevé et broyé dans un milieu RPMI à l'aide d'un Potter. Une partie du broyat est ensuite déposé sur cellules HFF en milieu DMEM supplémenté par 10% de sérum de veau foetal (FCS), 2 mM glutamine, 50 U/mL de pénicilline et 50 µg/mL de streptomycine. Les parasites sont ensuite récoltés et leur ADN génomique est extrait.

A partir de l'ADN génomique, des PCR ont été réalisées pour :
- vérifier la présence ou l'absence du gène *ncmic3* avec le jeu d'amorces de la PCR n°1: ORF NCmic3 F (SEQ ID NO: 7) et ORF NCmic3 R (SEQ ID NO: 8).
- vérifier la présence ou l'absence de la cassette *DHFR* avec le jeu d'amorce de la PCR n°2: ORF DHFR F (SEQ ID NO: 9) et ORF DHFR R (SEQ ID NO: 10).
- vérifier la présence ou l'absence du gène *ncmic1* avec le jeu d'amorces de la PCR n°3: stop Ncmic1 (SEQ ID NO: 39) et ORF NCmic1 R (SEQ ID NO: 25).
- vérifier la présence ou l'absence de la cassette *CATGFP* avec le jeu d'amorce de la PCR n°4: ORF CATGFP F3 (SEQ ID NO: 49) et stop CATGFP (SEQ ID NO: 42).

Les séquences des amorces et la taille des amplicons issus des différentes PCR figurent respectivement dans les Tableaux XVI et XVII ci-dessous.

**Tableaux XVI : Liste des amorces ayant servi à diagnostiquer les souris vaccinées par la souche Neo ncmic1-3 KO et les souris infectées par la souche NC-1 de N. caninum.**

| **Nom de l'amorce** | **Séquence 5' → 3'** | **n° de séquence** | **n° de PCR** |
|---|---|---|---|
| ORF NCmic3 F | TTTCCCTTCTAAACACAGTCG | SEQ ID NO: 7 | 1 |
| ORF NCmic3 R | CCTTCAGTGGTTCTCCATGAG T | SEQ ID NO: 8 | 1 |
| ORF DHFR F | CCTTCTCAGACAACGGGGTA | SEQ ID NO: 9 | 2 |
| ORF DHFR R | AGATCTTCACGCCCTTCTCA | SEQ ID NO: 10 | 2 |
| stop Ncmic1 | TTACAATTCAGATTCACCCG | SEQ ID NO: 39 | 3 |
| ORF NCmic1 R | TTCTCCAGGCACTCACCT | SEQ ID NO: 25 | 3 |
| ORF CATGFP F3 | TTCATCATGCCGTTTGTGAT | SEQ ID NO: 49 | 4 |
| stop CATGFP | TTAATCGAGCGGGTCCTGGT | SEQ ID NO: 42 | 4 |

**Tableaux XVII : Taille des amplicons obtenus en paires de bases des différentes PCR permettant le diagnostic différentiel entre des souris vaccinées par la souche Neo ncmic1-3 KO et les souris infectées par la souche NC-1 de N. caninum.**

| **n° de PCR** | **Neo *ncmic1-3* KO** | ***Neospora caninum* (NC-1)** |
|---|---|---|
| 1 | - | 850 |
| 2 | 504 | - |
| 3 | - | 716 |
| 4 | 875 | - |

Les amplicons obtenus par PCR à partir des échantillons issus de souris vaccinées et ceux obtenus à partir des échantillons issus de souris challengées par la souche NC-1 (Figure 14) sont conformes aux profils attendus et mettent en évidence :
- l'absence des gènes *ncmic3 et ncmic1* et la présence des cassettes *DHFR et CATGFP* pour les échantillons issus de souris vaccinées avec la souche mutante Neo *ncmic1-3* KO
- la présence des gènes *ncmic3 et ncmic1* et l'absence des cassettes *DHFR et CATGFP* pour les échantillons issus de souris infectés avec la souche NC-1 de *N. caninum.*

Ces résultats confirment la possibilité de différencier les animaux vaccinés des animaux infectés.

## Revendications

1. Souche mutante de *Neospora caninum* dans laquelle la fonction de la protéine NcMIC3 est supprimée par la délétion de tout le gène *ncmic3.*

2. Souche mutante de *Neospora caninum* selon la revendication 1, dans laquelle le gène de sélection *dhfr* codant pour la protéine DHFR est inséré par recombinaison homologue en lieu et place du gène *ncmic3.*

3. Souche mutante de *Neospora caninum* selon la revendication 1, dans laquelle la fonction de la protéine NcMIC1 est supprimée par la délétion de tout le gène *ncmic1.*

4. Souche mutante de *Neospora caninum* selon la revendication 3, dans laquelle le gène de sélection *dhfr* codant pour la protéine DHFR est inséré par recombinaison homologue en lieu et place du gène *ncmic3* et/ou le gène de sélection *cat-gfp* codant pour la protéine CAT-GFP est inséré par recombinaison homologue en lieu et place du gène *ncmic1.*

5. Composition pharmaceutique comprenant une souche mutante de *Neospora caninum* dans laquelle la fonction de la protéine NcMIC3 est supprimée selon la revendication 1 ou 2 et un véhicule pharmaceutiquement acceptable.

6. Composition pharmaceutique comprenant une souche mutante de *Neospora caninum* dans laquelle la fonction de la protéine NcMIC3 et la fonction de la protéine NcMIC1 sont supprimées selon la revendication 3 ou 4 et un véhicule pharmaceutiquement acceptable.

7. Composition vaccinale comprenant une souche mutante telle que définie selon la revendication 1 ou 3 et un véhicule pharmaceutiquement acceptable pour utilisation dans le traitement de la néosporose chez les animaux de compagnie, tels que les chiens et les chevaux et les animaux de rentes, tels que les ovins, les caprins, les bovins, les porcins, les camélidés et les cervidés.

8. Composition vaccinale comprenant une souche mutante telle que définie selon la revendication 2 ou 4 et un véhicule pharmaceutiquement acceptable pour utilisation dans le traitement de la néosporose chez les animaux de compagnie, tels que les chiens et les chevaux et les animaux de rentes, tels que les ovins, les caprins, les bovins, les porcins, les camélidés et les cervidés.

9. Composition vaccinale pour utilisation selon la revendication 7, pour une administration en une dose unitaire variant de 10² à 10⁹ tachyzoïtes d'une souche mutante telle que définie selon la revendication 1 ou 3.

10. Composition vaccinale pour utilisation selon la revendication 8, pour une administration en une dose unitaire variant de 10² à 10⁹ tachyzoïtes d'une souche mutante telle que définie selon la revendication 2 ou 4.

11. Procédé *in vitro* de diagnostic différentiel pour discriminer si un mammifère est un mammifère vacciné par une composition telle que définie selon la revendication 8 ou 10, ou un mammifère non vacciné, ledit procédé comprenant les étapes suivantes :
i) détermination de la présence d'anticorps anti-DHFR et/ou anti-CAT-GFP, et/ou,
détermination de la présence d'antigènes DHFR et/ou CAT-GFP,
ou
ii) détermination du niveau d'expression des gènes *dhfr* et/ou *cat-gfp*,
et/ou,
détermination de la présence des gènes *dhfr* et/ou *cat-gfp*,
dans un échantillon biologique du susdit mammifère,
la détermination de la présence d'anticorps anti-DHFR et/ou anti-CAT-GFP, et/ou la détermination de la présence d'antigènes DHFR et/ou CAT-GFP, ou la détermination du niveau d'expression des gènes *dhfr* et/ou *cat-gfp*, et/ou la détermination de la présence des gènes *dhfr* et/ou *cat-gfp*, signifiant que ledit mammifère est un mammifère vacciné.

## Patentansprüche

1. Mutantenstamm von *Neospora caninum*, wobei die Funktion des Proteins NcMIC3 durch die Deletion des gesamten Gens *ncmic3* supprimiert wird.

2. Mutantenstamm von *Neospora caninum* nach Anspruch 1, wobei das Selektionsgen *dhfr*, das für das Protein DHFR codiert, durch homologe Rekombination anstelle des Gens *ncmic3* insertiert wird.

3. Mutantenstamm von *Neospora caninum* nach Anspruch 1, wobei die Funktion des Proteins McMIC1 durch die Deletion des gesamten Gens *ncmic1* supprimiert wird.

4. Mutantenstamm von *Neospora caninum* nach Anspruch 3, wobei das Selektionsgen *dhfr,* das für das Protein DHFR codiert, durch homologe Rekombination anstelle des Gens *ncmic3* insertiert wird, und/oder das Selektionsgen *cat-gfp*, das für das Protein CAT-GFP codiert, durch homologe Rekombination anstelle des Gens *ncmic1* insertiert wird.

5. Pharmazeutische Zusammensetzung, umfassend einen Mutantenstamm von *Neospora caninum*, wobei die Funktion des Proteins NcMIC3 nach Anspruch 1 oder 2 supprimiert wird, und einen pharmazeutisch annehmbaren Träger.

6. Pharmazeutische Zusammensetzung, umfassend einen Mutantenstamm von *Neospora caninum*, wobei die Funktion des Proteins NcMIC3 und die Funktion des Proteins NcMIC1 nach Anspruch 3 oder 4 supprimiert werden, und einen pharmazeutisch annehmbaren Träger.

7. Impfstoffzusammensetzung, umfassend einen Mutantenstamm, wie nach Anspruch 1 oder 3 definiert, und einen pharmazeutisch annehmbaren Träger, welche geeignet ist zur Verwendung bei der Behandlung von Neosporose bei Haustieren, wie Hunden und Pferden, und Nutztieren, wie Schafen, Ziegen, Rindern, Schweinen, Kamelen und Rotwild.

8. Impfstoffzusammensetzung, umfassend einen Mutantenstamm, wie nach Anspruch 2 oder 4 definiert, und einen pharmazeutisch annehmbaren Träger, welche geeignet ist zur Verwendung bei der Behandlung von Neosporose bei Haustieren, wie Hunden und Pferden, und Nutztieren, wie Schafen, Ziegen, Rindern, Schweinen, Kamelen und Rotwild.

9. Impfstoffzusammensetzung zur Verwendung nach Anspruch 7, zur Verabreichung in einer Einheitsdosis, die von 10² bis 10⁹ Tachyzoiten eines Mutantenstamms variiert, wie nach Anspruch 1 oder 3 definiert.

10. Impfstoffzusammensetzung zur Verwendung nach Anspruch 8, zur Verabreichung in einer Einheitsdosis, die von 10² bis 10⁹ Tachyzoiten eines Mutantenstamms variiert, wie nach Anspruch 2 oder 4 definiert.

11. *In vitro* Verfahren zur Differentialdiagnose, um zu diskriminieren, ob ein Säuger ein Säuger, der durch eine Zusammensetzung, wie nach Anspruch 8 oder 10 definiert, geimpft wurde, oder ein ungeimpfter Säuger ist, wobei das Verfahren die folgenden Schritte umfasst:
(i) Bestimmung des Vorliegens von anti-DHFR- und/oder anti-CAT-GFP-Antikörpern, und/oder
Bestimmung des Vorliegens von DHFR- und/oder CAT-GFP-Antigenen, oder
(ii) Bestimmung des Expressionsniveaus der Gene *dhfr* und/oder *cat-gfp*, und/oder
Bestimmung des Vorliegens der Gene *dhfr* und/oder *cat-gfp*,
in einer biologischen Probe des Säugers,
wobei die Bestimmung des Vorliegens von anti-DHFR- und/oder anti-CAT-GFP-Antikörpern, und/oder die Bestimmung des Vorliegens von DHFR- und/oder CAT-GFP-Antigenen, oder die Bestimmung des Expressionsniveaus der Gene *dhfr* und/oder *cat-gfp*, und/oder die Bestimmung des Vorliegens der Gene *dhfr* und/oder cat-*gfp*, bedeuten, dass der Säuger ein geimpfter Säuger ist.

## Claims

1. Mutant strain of *Neospora caninum*, wherein the function of the NcMIC3 protein is suppressed by the deletion of the whole of the *ncmic3* gene.

2. Mutant strain of *Neospora caninum* according to claim 1, wherein the *dhfr* selection gene encoding the DHFR protein is inserted by homologous recombination in place of the *ncmic3* gene.

3. Mutant strain of *Neospora caninum* according to claim 1, wherein the function of the NcMIC1 protein is suppressed by the deletion of the whole of the *ncmic1* gene.

4. Mutant strain of *Neospora caninum* according to claim 3, wherein the *dhfr* selection gene encoding the DHFR protein is inserted by homologous recombination in place of the *ncmic3* gene and/or the *cat-gfp* selection gene encoding the CAT-GFP protein is inserted by homologous recombination in place of the *ncmic1* gene.

5. Pharmaceutical composition comprising a mutant strain of *Neospora caninum* wherein the function of the NcMIC3 protein is suppressed according to claim 1 or 2 and a pharmaceutically acceptable vehicle.

6. Pharmaceutical composition comprising a mutant strain of *Neospora caninum* wherein the function of the NcMIC3 protein and the function of the NcMIC1 protein are suppressed according to claim 3 or 4 and a pharmaceutically acceptable vehicle.

7. Vaccine composition comprising a mutant strain as defined according to claim 1 or 3 and a pharmaceutically acceptable vehicle for the use in the treatment of neosporosis in pet animals, such as dogs and horses and farm animals, such as ovines, caprins, bovines, porcines, camelids and cervids.

8. Vaccine composition comprising a mutant strain as defined according to claim 2 or 4 and a pharmaceutically acceptable vehicle for the use in the treatment of neosporosis in pet animals, such as dogs and horses and farm animals, such as ovines, caprins, bovines, porcines, camelids and cervids.

9. Vaccine composition for its use according to claim 7, which is administered in a unit dose varying from 10² to 10⁹ tachyzoïtes of a mutant strain as defined according to claim 1 or 3.

10. Vaccine composition for its use according to claim 8, which is administered in a unit dose varying from 10² to 10⁹ tachyzoïtes of a mutant strain as defined according to claim 2 or 4.

11. *In vitro* method of differential diagnostics for discriminating if a mammal is a vaccinated mammal with the compositions as defined according to claim 8 or 10 from an unvaccinated mammal, said method comprising the following steps:
i) determination of the presence of anti-DHFR and/or anti-CAT-GFP antibodies,
and/or,
determination of the presence of DHFR and/or CAT-GFP antigen,
or
ii) determination of the expression level of the genes *dhfr* and/or *cat-gfp*, and/or,
determination of the presence of the genes *dhfr* and/or *cat-gfp*,
in a biological sample from the aforesaid mammal,
the determination of the presence of anti-DHFR and/or anti-CAT-GFP antibodies, and/or the determination of the presence of DHFR and/or CAT-GFP antigen, or the determination of the expression level of the genes *dhfr* and/or *cat-gfp*, and/or the determination of the presence of the genes *dhfr* and/or *cat-gfp*, meaning that said mammal is a vaccinated mammal.


### SEQUENCE LISTING

<110> VitamFero
<120> SOUCHES MUTANTES DE NEOSPORA ET LEURS UTILISATIONS
<130> WOB 11 CA ITA NEKO
<160> 49
<170> PatentIn version 3.5
<210> 1
   <211> 34
   <212> DNA
   <213> Artificial Sequence
<220>
   <223> 5 HR NCmic3 F KpnI
<400> 1
   cgcggtaccc atgtgaatat gctttaaccg tgac 34
<210> 2
   <211> 34
   <212> DNA
   <213> Artificial Sequence
<220>
   <223> 5 HR NCmic3 R ClaI
<400> 2
   cgcatcgatg agctataacc cttggaaatg actc 34
<210> 3
   <211> 31
   <212> DNA
   <213> Artificial Sequence
<220>
   <223> 3 HR NCmic3 F XbaI
<400> 3
   cgctctagac atgctgatga agaagggaag t 31
<210> 4
   <211> 34
   <212> DNA
   <213> Artificial Sequence
<220>
   <223> 3 HR NCmic3 R NotI
<400> 4
   cgcgcggccg ctctctcctg aagtcttcga gacc 34
<210> 5
   <211> 25
   <212> DNA
   <213> Artificial Sequence
<220>
   <223> HR NCmic3 F
<400> 5
   gtcatcgacc gccggaacta gtagt 25
<210> 6
   <211> 26
   <212> DNA
   <213> Artificial Sequence
<220>
   <223> HR NCmic3 R
<400> 6
   gcagaggttc tgcgtatcta acacgg 26
<210> 7
   <211> 21
   <212> DNA
   <213> Artificial Sequence
<220>
   <223> ORF NCmic3 F
<400> 7
   tttcccttct aaacacagtc g 21
<210> 8
   <211> 22
   <212> DNA
   <213> Artificial Sequence
<220>
   <223> ORF NCmic3 R
<400> 8
   ccttcagtgg ttctccatga gt 22
<210> 9
   <211> 20
   <212> DNA
   <213> Artificial Sequence
<220>
   <223> ORF DHFR F
<400> 9
   ccttctcaga caacggggta 20
<210> 10
   <211> 20
   <212> DNA
   <213> Artificial Sequence
<220>
   <223> ORF DHFR R
<400> 10
   agatcttcac gcccttctca 20
<210> 11
   <211> 24
   <212> DNA
   <213> Artificial Sequence
<220>
   <223> Integ NCmic3 F
<400> 11
   gaaagtgtca gtggtagaga ctgc 24
<210> 12
   <211> 27
   <212> DNA
   <213> Artificial Sequence
<220>
   <223> ORF NCmic3 R2
<400> 12
   ccttcactcg agatcgcgca aatgagc 27
<210> 13
   <211> 23
   <212> DNA
   <213> Artificial Sequence
<220>
   <223> ORF DHFR R2
<400> 13
   ggacctctgt acgagacatg ccg 23
<210> 14
   <211> 24
   <212> DNA
   <213> Artificial Sequence
<220>
   <223> Integ NCmic3 R
<400> 14
   tgtttacagg tgatccagaa aagg 24
<210> 15
   <211> 20
   <212> DNA
   <213> Artificial Sequence
<220>
   <223> ORF NCmic3 F2
<400> 15
   gaattttggg acaggggaat 20
<210> 16
   <211> 25
   <212> DNA
   <213> Artificial Sequence
<220>
   <223> ORF DHFR F2
<400> 16
   gtctctcgtt ttcctctctt ttcgg 25
<210> 17
   <211> 33
   <212> DNA
   <213> Artificial Sequence
<220>
   <223> 3 HR NCmic1 F KpnI
<400> 17
   cgcggtacca ggcagaagta aagaaggttc ctc 33
<210> 18
   <211> 30
   <212> DNA
   <213> Artificial Sequence
<220>
   <223> 3 HR NCmic1 R HindIII
<400> 18
   cgcaagcttt gatcacgcaa gaaaagaagc 30
<210> 19
   <211> 32
   <212> DNA
   <213> Artificial Sequence
<220>
   <223> 5 HR NCmic1 F BamHI
<400> 19
   cgcggatccc atttgtagat acggttgcac ac 32
<210> 20
   <211> 33
   <212> DNA
   <213> Artificial Sequence
<220>
   <223> 5 HR NCmic1 R NotI
<400> 20
   cgcgcggccg cacattcaga cggcagaact ctg 33
<210> 21
   <211> 20
   <212> DNA
   <213> Artificial Sequence
<220>
   <223> Integ NCmic1 F
<400> 21
   ccgagcaagt tagcaagtcc 20
<210> 22
   <211> 20
   <212> DNA
   <213> Artificial Sequence
<220>
   <223> ORF CATGFP R
<400> 22
   ccgtttggtg gatgtcttct 20
<210> 23
   <211> 20
   <212> DNA
   <213> Artificial Sequence
<220>
   <223> ORF CATGFP F
<400> 23
   gcatcgactt caaggaggac 20
<210> 24
   <211> 20
   <212> DNA
   <213> Artificial Sequence
<220>
   <223> Integ NCmic1 R
<400> 24
   cttgtccgtc acatcgtttg 20
<210> 25
   <211> 20
   <212> DNA
   <213> Artificial Sequence
<220>
   <223> ORF NCmic1 R
<400> 25
   ttctccaggc actcacctct 20
<210> 26
   <211> 20
   <212> DNA
   <213> Artificial Sequence
<220>
   <223> ORF NCmic1 F
<400> 26
   agcttccaac aacgagagga 20
<210> 27
   <211> 20
   <212> DNA
   <213> Artificial Sequence
<220>
   <223> ORF NCmic1 F2
<400> 27
   cccaggatat cgtttgttgc 20
<210> 28
   <211> 20
   <212> DNA
   <213> Artificial Sequence
<220>
   <223> ORF NCmic1 R2
<400> 28
   cttctgatgc acggaactga 20
<210> 29
   <211> 20
   <212> DNA
   <213> Artificial Sequence
<220>
   <223> ORF CATGFP F2
<400> 29
   cctgaagttc atctgcacca 20
<210> 30
   <211> 20
   <212> DNA
   <213> Artificial Sequence
<220>
   <223> ORFCATGFP R2
<400> 30
   gtagtggttg tcgggcagca 20
<210> 31
   <211> 23
   <212> DNA
   <213> Artificial Sequence
<220>
   <223> NC5 FA
<400> 31
   cccagtgcgt ccaatcctgt aac 23
<210> 32
   <211> 24
   <212> DNA
   <213> Artificial Sequence
<220>
   <223> NC5 RA
<400> 32
   ctcgccagtc aacctacgtc ttct 24
<210> 33
   <211> 20
   <212> DNA
   <213> Artificial Sequence
<220>
   <223> NC5 FB
<400> 33
   taatctcccc cgtcatcagt 20
<210> 34
   <211> 19
   <212> DNA
   <213> Artificial Sequence
<220>
   <223> NC5 RB
<400> 34
   gggtgaaccg agggagttg 19
<210> 35
   <211> 20
   <212> DNA
   <213> Artificial Sequence
<220>
   <223> ATG Ncmic1
<400> 35
   atgggccagt cggtggtttt 20
<210> 36
   <211> 20
   <212> DNA
   <213> Artificial Sequence
<220>
   <223> ATG Ncmic3
<400> 36
   atgcgtggcg gggcgtccgc 20
<210> 37
   <211> 19
   <212> DNA
   <213> Artificial Sequence
<220>
   <223> ATG DHFR
<400> 37
   atgcagaaac cggtgtgtc 19
<210> 38
   <211> 22
   <212> DNA
   <213> Artificial Sequence
<220>
   <223> ATG CATGFP
<400> 38
   atgcatgaga aaaaaatcac tg 22
<210> 39
   <211> 20
   <212> DNA
   <213> Artificial Sequence
<220>
   <223> stop Ncmic1
<400> 39
   ttacaattca gattcacccg 20
<210> 40
   <211> 22
   <212> DNA
   <213> Artificial Sequence
<220>
   <223> stop Ncmic3
<400> 40
   ttatcgagcc gttccgcatt tg 22
<210> 41
   <211> 21
   <212> DNA
   <213> Artificial Sequence
<220>
   <223> stop DHFR
<400> 41
   ctagacagcc atctccatct g 21
<210> 42
   <211> 20
   <212> DNA
   <213> Artificial Sequence
<220>
   <223> stop CATGFP
<400> 42
   ttaatcgagc gggtcctggt 20
<210> 43
   <211> 21
   <212> DNA
   <213> Artificial Sequence
<220>
   <223> Olig 1
<400> 43
   cagatggaga tggctgtcta g 21
<210> 44
   <211> 20
   <212> DNA
   <213> Artificial Sequence
<220>
   <223> Olig 2
<400> 44
   cgctttcgtt ctgattgaca 20
<210> 45
   <211> 20
   <212> DNA
   <213> Artificial Sequence
<220>
   <223> Olig 3
<400> 45
   aaaaccaccg actggcccat 20
<210> 46
   <211> 20
   <212> DNA
   <213> Artificial Sequence
<220>
   <223> Olig 4
<400> 46
   tcctctcgtt gttggaagct 20
<210> 47
   <211> 20
   <212> DNA
   <213> Artificial Sequence
<220>
   <223> Olig 5
<400> 47
   tagcacggga aaggattgac 20
<210> 48
   <211> 19
   <212> DNA
   <213> Artificial Sequence
<220>
   <223> Olig 6
<400> 48
   caagatccgc cacaacatc 19
<210> 49
   <211> 20
   <212> DNA
   <213> Artificial Sequence
<220>
   <223> ORF CATGFP F3
<400> 49
   ttcatcatgc cgtttgtgat 20